# EUROPEAN PATENT SPECIFICATION

(11) **EP 3 796 850 B1**
(45) Date of publication and mention of the grant of the patent: **09.10.2024**
(21) Application number: 19730063.5
(22) Date of filing: 23.05.2019
(51) Int. Cl.: A61B 17/12, A61B 17/00

(54) **OCCLUSIVE DEVICE WITH EXPANDABLE MEMBER**
VERSCHLUSSVORRICHTUNG MIT EXPANDIERBAREM ELEMENT
DISPOSITIF OCCLUSIF POURVU D'UN ÉLÉMENT DILATABLE

(30) Priority: 23.05.2018 US 201862675593 P
(43) Date of publication of application: 31.03.2021
(73) Proprietor: Boston Scientific Scimed, Inc., Maple Grove, MN 55311 (US)
(72) Inventor: INOUYE, Joshua Mark, Maple Grove, Minnesota 55369 (US); ONUSHKO, David John, Maple Grove, Minnesota 5369 (US); ANDERSON, James M., Corcoran, Minnesota 55357 (US); MEREGOTTE, Jose A., Blaine, Minnesota 55449 (US); RAAB, David, Roseville, Minnesota 55113 (US); LARSEN, Steven R., Lino Lakes, Minnesota 55110 (US); EDGELL, John M., Plymouth, Minnesota 55441 (US); TASSONI, Nicholas Lee, Andover, Minnesota 55303 (US)
(74) Representative: Vossius & Partner Patentanwälte Rechtsanwälte mbB
(86) International application number: PCT/US2019/033698
(87) International publication number: WO 2019/226868

(56) References cited:
- WO-A1-2015/164836
- WO-A1-2018/017935
- CN-A- 106 859 722
- US-A1- 2002 082 638
- US-A1- 2005 015 140
- US-A1- 2011 295 226
- US-A1- 2014 039 536
- US-A1- 2014 100 596
- US-A1- 2016 287 259
- US-A1- 2019 223 883

## Description

### BACKGROUND

The left atrial appendage (LAA) is a small organ attached to the left atrium of the heart as a pouch-like extension. In patients suffering from atrial fibrillation, the left atrial appendage may not properly contract with the left atrium, causing stagnant blood to pool within its interior, which can lead to the undesirable formation of thrombi within the left atrial appendage. Thrombi forming in the left atrial appendage may break loose from this area and enter the blood stream. Thrombi that migrate through the blood vessels may eventually plug a smaller vessel downstream and thereby contribute to stroke or heart attack. Clinical studies have shown that the majority of blood clots in patients with atrial fibrillation are found in the left atrial appendage. As a treatment, medical devices have been developed which are positioned in the left atrial appendage and deployed to close off the ostium of the left atrial appendage. Over time, the exposed surface(s) spanning the ostium of the left atrial appendage becomes covered with tissue (a process called endothelization), effectively removing the left atrial appendage from the circulatory system and reducing or eliminating the number of thrombi which may enter the blood stream from the left atrial appendage. A continuing need exists for improved medical devices and methods to control thrombus formation within the left atrial appendage of patients suffering from atrial fibrillation.

US 2016/287259 A1 relates to medical devices and methods for forming the medical devices. An occlusion balloon comprises an outer balloon member, and an inner balloon member having an inner wall and an outer wall and extending through at least a portion of the outer balloon member. When forces acting on the inner wall of the inner balloon member equal forces acting on the outer wall of the inner balloon member, the inner balloon member defines a lumen.

US 2014/039536 A1 relates to a medical device including a balloon member, a port that couples a compartment interior of the balloon member to a region exterior of the balloon member, and a tubular member that defines a lumen. A distal end of the tubular member is attached to an internal surface of the balloon member within the compartment so that a portion of the internal surface of the balloon member provides a seal at a distal end of the lumen. The tubular member passes through the port, and the proximal end of the tubular member is configured to remain exterior of the compartment. A delivery of a sufficient amount of a filling material into the lumen of the tubular member causes a length of the tubular member to pass through the port and into the balloon compartment.

WO 2015/164836 A1 relates to an occlusion device. The exemplary occlusion device includes a cap chamber and a bulb chamber for occluding a left atrial appendage (LAA). After delivery to the LAA, the cap chamber and the bulb chamber are each inflated via various amounts of fluid(s) to occlude the LAA.

US 2002/082638 A1 relates to a process and apparatus for occluding a vascular site. A catheter having a highly distensible occlusion balloon detachably mounted at a distal end thereof; is provided to the vascular site and at least partially filled with an aqueous balloon inflation fluid which allows the balloon placement to be imaged. The balloon is porous to the aqueous inflation fluid and the inflation fluid is gradually displaced through the balloon pores by a liquid filling composition which is solidifiable on contact with the aqueous inflation fluid to produce a mass to which the balloon is nonporous. The filling composition is injected to bring the balloon wall into contact with the vessel wall at the vascular site. The balloon is detached from the catheter after the filling composition has at least substantially solidified and the catheter removed from the body.

US 2014/100596 A1 relates to a medical device for reducing the volume of a left atrial appendage. The device includes an elongate shaft having a distal portion, and a volume-reducing means expandable from a collapsed to an expanded state, the volume-reducing means being releasably attached to the distal portion. The volume-reducing means may include an actuatable frame and an impermeable covering disposed over the frame. The volume-reducing means may be sized to fit within the LAA in the expanded state while maintaining an open fluid flow path from a distal region through the ostium of the LAA. A medical device may include a second volume-reducing means to be placed within and substantially occlude a distalmost region of the LAA. A method may include inserting a volume-reducing means into the LAA, expanding the volume-reducing means, and positioning the volume-reducing means such that an open fluid flow path is maintained through an entire cycle of the heart.

### SUMMARY

The invention for which protection is sought is defined by the independent claim. The dependent claims concern particular embodiments.

This disclosure provides design, material, manufacturing method, and use alternatives for medical devices. An example medical device for occluding the left atrial appendage includes an expandable member having a first end region and a second end region. The expandable member may comprise at least one inflation cavity and at least one valve member configured to selectively seal the inflation cavity. A first inflation media and a second inflation media may be disposed within the at least one inflation cavity, the second inflation media different than the first inflation media. The expandable member may be configured to expand and seal the opening of the left atrial appendage.

Alternatively or additionally to any of the examples above, in another example, the second inflation media may be absorbed within the first inflation media.

Alternatively or additionally to any of the examples above, in another example, the first inflation media may comprise a hydrogel and the second inflation media may comprise saline.

Alternatively or additionally to any of the examples above, in another example, the medical device may further comprise a release mechanism disposed within the at least one valve member and configured to be releasably coupled to a delivery system.

Alternatively or additionally to any of the examples above, in another example, the release mechanism may be configured to form an interlocked configuration with a mating release mechanism on the delivery system.

Alternatively or additionally to any of the examples above, in another example, a securement member may be configured to be slidably disposed within the release mechanism, the securement member actuatable between an interlocked position and a released position.

Alternatively or additionally to any of the examples above, in another example, the securement member may comprise an inflation lumen for delivering the first and the second inflation media to the inflation cavity.

Alternatively or additionally to any of the examples above, in another example, the expandable member may comprise an outer expandable member having an outer inflation cavity and a valve member configured to selectively seal the outer inflation cavity and an inner expandable member having an inner inflation cavity and a valve member configured to selectively seal the inner inflation cavity.

Alternatively or additionally to any of the examples above, in another example, the outer expandable member may be configured to receive the first inflation media and the inner expandable member may be configured to receive the second inflation media.

Alternatively or additionally to any of the examples above, in another example, the first inflation media may be a thermally reversible copolymer.

Alternatively or additionally to any of the examples above, in another example, the thermally reversible copolymer may be a liquid at temperatures in the range of about 20 to about 25 °C and a gel at temperatures in the range of about 36 to about 37.5 °C.

Alternatively or additionally to any of the examples above, in another example, the second inflation media may be delivered at a temperature less than the transition temperature of the thermally reversible copolymer.

Alternatively or additionally to any of the examples above, in another example, the thermally reversible copolymer may be a polyethylene glycol-poly(lactic-*co-*glycolic acid)-polyethylene glycol or a polyethylene glycol-polycaprolactone-polyethylene glycol.

Alternatively or additionally to any of the examples above, in another example, the medical device may further comprise a plurality of apertures extending from an inner surface to an outer surface of the outer expandable member.

Alternatively or additionally to any of the examples above, in another example, an inflation media may be configured to weep from the plurality of apertures.

In another example, a medical device for occluding the left atrial appendage may comprise an expandable member having a first end region and a second end region. The expandable member may comprise at least one inflation cavity and at least one valve member configured to selectively seal the inflation cavity. A first inflation media and a second inflation media may disposed within the at least one inflation cavity. The second inflation media may be different than the first inflation media. The expandable member may be configured to expand and seal the opening of the left atrial appendage.

Alternatively or additionally to any of the examples above, in another example, the second inflation media may be absorbed within the first inflation media.

Alternatively or additionally to any of the examples above, in another example, the first inflation media may comprise a hydrogel and the second inflation media may comprise saline.

Alternatively or additionally to any of the examples above, in another example, the medical device may further comprise a release mechanism disposed within the at least one valve member and configured form an interlocked configuration with a mating release mechanism on the delivery system.

Alternatively or additionally to any of the examples above, in another example, the medical device may further comprise a securement member having an inflation lumen for delivering the first and second inflation media to the inflation cavity, the securement member configured to be slidably disposed within the release mechanism and actuatable between an interlocked position and a released position.

Alternatively or additionally to any of the examples above, in another example, the expandable member may comprise an outer expandable member having an outer inflation cavity and a valve member configured to selectively seal the outer inflation cavity and an inner expandable member having an inner inflation cavity and a valve member configured to selectively seal the inner inflation cavity.

Alternatively or additionally to any of the examples above, in another example, the outer expandable member may be configured to receive the first inflation media and the inner expandable member may be configured to receive the second inflation media.

Alternatively or additionally to any of the examples above, in another example, the first inflation media may be a thermally reversible copolymer which may be a liquid at temperatures in the range of about 20 to about 25 °C and a gel at temperatures in the range of about 36 to about 37.5 °C.

In another example, a medical device for occluding the left atrial appendage may comprise an expandable member having a first end region and a second end region. The expandable member may comprise an outer expandable member having an outer inflation cavity and a valve member configured to selectively seal the outer inflation cavity and an inner expandable member disposed within the outer inflation cavity and coupled to the outer expandable member. The inner expandable member may have an inner inflation cavity and a valve member configured to selectively seal the inner inflation cavity. The expandable member may be configured to expand and seal the opening of the left atrial appendage.

Alternatively or additionally to any of the examples above, in another example, the outer expandable member may be configured to receive a first inflation media and the inner expandable member may be configured to receive a second inflation media different from the first.

Alternatively or additionally to any of the examples above, in another example, the first inflation media may be a thermally reversible copolymer.

Alternatively or additionally to any of the examples above, in another example, the thermally reversible copolymer may be a liquid at temperatures in the range of about 20 to about 25 °C and a gel at temperatures in the range of about 36 to about 37.5 °C and transitions from the liquid to the gel at a transition temperature.

Alternatively or additionally to any of the examples above, in another example, the second inflation media may be delivered at a temperature less than the transition temperature of the thermally reversible copolymer.

Alternatively or additionally to any of the examples above, in another example, the thermally reversible copolymer may be a polyethylene glycol-poly(lactic-*co-*glycolic acid)-polyethylene glycol or a polyethylene glycol-polycaprolactone-polyethylene glycol.

Alternatively or additionally to any of the examples above, in another example, the medical device may further comprise a plurality of apertures extending from an inner surface to an outer surface of the outer expandable member, the plurality of apertures configured to allow the first inflation media to weep from the outer inflation cavity and through the plurality of apertures.

In another example, a medical device system for occluding the left atrial appendage may comprise an expandable member having a first end region and a second end region and a delivery and inflation system. The expandable member may comprise an inflation cavity, a valve member configured to selectively seal the inflation cavity and a first portion of a release mechanism disposed within the valve member. The delivery and inflation system may comprise an outer elongate shaft defining a lumen, a second portion of the release mechanism coupled to a distal end of the elongate shaft, and a securement member slidably disposed within the lumen of the outer elongate shaft and the second portion of the release mechanism and defining a lumen configured to be fluidly coupled to the inflation cavity.

Alternatively or additionally to any of the examples above, in another example, the securement member may be actuatable between an interlocked configuration and a released configuration to releasably secure the expandable member to the delivery and inflation system.

Alternatively or additionally to any of the examples above, in another example, the inflation cavity may be configured to receive a first inflation media and a second inflation media different from the first inflation media

Alternatively or additionally to any of the examples above, in another example, the first inflation media may comprise a hydrogel and the second inflation media may comprise saline.

Alternatively or additionally to any of the examples above, in another example, the second inflation media may be absorbed within the first inflation media.

The above summary of some embodiments is not intended to describe each disclosed embodiment or every implementation of the present disclosure. The Figures, and Detailed Description, which follow, more particularly exemplify these embodiments

### BRIEF DESCRIPTION OF THE DRAWINGS

The disclosure may be more completely understood in consideration of the following detailed description of various embodiments in connection with the accompanying drawings, in which:
FIG. 1 is a plan view of an example occlusive implant;
FIG. 2 illustrates a bottom view of the example occlusive implant shown in FIG. 1;
FIG. 3 illustrates a cross-sectional view along line 3-3 of FIG. 2;
FIG. 4 illustrates a partial cross-sectional view of another example occlusive implant in first stage of expansion with a delivery and/or inflation system;
FIG. 5A illustrates partial cross-sectional view of the occlusive implant FIG. 4 in a second stage of expansion with a delivery and/or inflation system;
FIG. 5B illustrates another partial cross-sectional view of the occlusive implant FIG. 4 in a second stage of expansion;
FIG. 6 illustrates a cross-sectional view an illustrative catheter for use with an occlusive implant;
FIG. 7 illustrates a cross-sectional view of another example occlusive implant;
FIG. 8 illustrates a cross-sectional view of the occlusive implant of FIG. 7 with a delivery system;
FIG. 9 illustrates a cross-sectional view of another example occlusive implant;
FIG. 10 illustrates a cross-sectional view of the occlusive implant of FIG. 8 with a delivery system in first stage of expansion;
FIG. 11 illustrates a cross-sectional view of the occlusive implant of FIG. 8 with a delivery system in second stage of expansion;
FIG. 12 illustrates a cross-sectional view of the occlusive implant of FIG. 8 with a delivery system in third stage of expansion;
FIG. 13 illustrates a side view of another example occlusive implant positioned within an opening of the left atrial appendage; and
FIGS. 14-19 illustrate an example occlusive implant being positioned within an opening of the left atrial appendage.

While aspects of the disclosure are amenable to various modifications and alternative forms, specifics thereof have been shown by way of example in the drawings and will be described in detail. It should be understood, however, that the intention is not to limit aspects of the disclosure to the particular embodiments described.

### DETAILED DESCRIPTION

The following description should be read with reference to the drawings, which are not necessarily to scale, wherein like reference numerals indicate like elements throughout the several views. The detailed description and drawings are intended to illustrate but not limit the claimed disclosure. Those skilled in the art will recognize that the various elements described and/or shown may be arranged in various combinations and configurations without departing from the scope of the disclosure. The detailed description and drawings illustrate example embodiments of the claimed disclosure. However, in the interest of clarity and ease of understanding, while every feature and/or element may not be shown in each drawing, the feature(s) and/or element(s) may be understood to be present regardless, unless otherwise specified.

For the following defined terms, these definitions shall be applied, unless a different definition is given in the claims or elsewhere in this specification.

All numeric values are herein assumed to be modified by the term "about," whether or not explicitly indicated. The term "about", in the context of numeric values, generally refers to a range of numbers that one of skill in the art would consider equivalent to the recited value (e.g., having the same function or result). In many instances, the term "about" may include numbers that are rounded to the nearest significant figure. Other uses of the term "about" (e.g., in a context other than numeric values) may be assumed to have their ordinary and customary definition(s), as understood from and consistent with the context of the specification, unless otherwise specified.

The recitation of numerical ranges by endpoints includes all numbers within that range, including the endpoints (e.g., 1 to 5 includes 1, 1.5, 2, 2.75, 3, 3.80, 4, and 5).

Although some suitable dimensions, ranges, and/or values pertaining to various components, features and/or specifications are disclosed, one of skill in the art, incited by the present disclosure, would understand desired dimensions, ranges, and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise. It is to be noted that in order to facilitate understanding, certain features of the disclosure may be described in the singular, even though those features may be plural or recurring within the disclosed embodiment(s). Each instance of the features may include and/or be encompassed by the singular disclosure(s), unless expressly stated to the contrary. For simplicity and clarity purposes, not all elements of the disclosure are necessarily shown in each figure or discussed in detail below. However, it will be understood that the following discussion may apply equally to any and/or all of the components for which there are more than one, unless explicitly stated to the contrary. Additionally, not all instances of some elements or features may be shown in each figure for clarity.

Relative terms such as "proximal", "distal", "advance", "retract", variants thereof, and the like, may be generally considered with respect to the positioning, direction, and/or operation of various elements relative to a user/operator/manipulator of the device, wherein "proximal" and "retract" indicate or refer to closer to or toward the user and "distal" and "advance" indicate or refer to farther from or away from the user. In some instances, the terms "proximal" and "distal" may be arbitrarily assigned in an effort to facilitate understanding of the disclosure, and such instances will be readily apparent to the skilled artisan. Other relative terms, such as "upstream", "downstream", "inflow", and "outflow" refer to a direction of fluid flow within a lumen, such as a body lumen, a blood vessel, or within a device.

The term "extent" may be understood to mean a greatest measurement of a stated or identified dimension, unless the extent or dimension in question is preceded by or identified as a "minimum", which may be understood to mean a smallest measurement of the stated or identified dimension. For example, "outer extent" may be understood to mean a maximum outer dimension, "radial extent" may be understood to mean a maximum radial dimension, "longitudinal extent" may be understood to mean a maximum longitudinal dimension, etc. Each instance of an "extent" may be different (e.g., axial, longitudinal, lateral, radial, circumferential, etc.) and will be apparent to the skilled person from the context of the individual usage. Generally, an "extent" may be considered a greatest possible dimension measured according to the intended usage, while a "minimum extent" may be considered a smallest possible dimension measured according to the intended usage. In some instances, an "extent" may generally be measured orthogonally within a plane and/or cross-section, but may be, as will be apparent from the particular context, measured differently - such as, but not limited to, angularly, radially, circumferentially (e.g., along an arc), etc.

The terms "monolithic" and "unitary" shall generally refer to an element or elements made from or consisting of a single structure or base unit/element. A monolithic and/or unitary element shall exclude structure and/or features made by assembling or otherwise joining multiple discrete elements together.

It is noted that references in the specification to "an embodiment", "some embodiments", "other embodiments", etc., indicate that the embodiment(s) described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it would be within the knowledge of one skilled in the art to effect the particular feature, structure, or characteristic in connection with other embodiments, whether or not explicitly described, unless clearly stated to the contrary. That is, the various individual elements described below, even if not explicitly shown in a particular combination, are nevertheless contemplated as being combinable or arrangeable with each other to form other additional embodiments or to complement and/or enrich the described embodiment(s), as would be understood by one of ordinary skill in the art.

For the purpose of clarity, certain identifying numerical nomenclature (e.g., first, second, third, fourth, etc.) may be used throughout the description and/or claims to name and/or differentiate between various described and/or claimed features. It is to be understood that the numerical nomenclature is not intended to be limiting and is exemplary only. In some embodiments, alterations of and deviations from previously-used numerical nomenclature may be made in the interest of brevity and clarity. That is, a feature identified as a "first" element may later be referred to as a "second" element, a "third" element, etc. or may be omitted entirely, and/or a different feature may be referred to as the "first" element. The meaning and/or designation in each instance will be apparent to the skilled practitioner.

The occurrence of thrombi in the left atrial appendage (LAA) during atrial fibrillation may be due to stagnancy of blood pooling in the LAA. The pooled blood may still be pulled out of the left atrium by the left ventricle, however less effectively due to the irregular contraction of the left atrium caused by atrial fibrillation. Therefore, instead of an active support of the blood flow by a contracting left atrium and left atrial appendage, filling of the left ventricle may depend primarily or solely on the suction effect created by the left ventricle. However, the contraction of the left atrial appendage may not be in sync with the cycle of the left ventricle. For example, contraction of the left atrial appendage may be out of phase up to 180 degrees with the left ventricle, which may create significant resistance to the desired flow of blood. Further still, most left atrial appendage geometries are complex and highly variable, with large irregular surface areas and a narrow ostium or opening compared to the depth of the left atrial appendage. These aspects as well as others, taken individually or in various combinations, may lead to high flow resistance of blood out of the left atrial appendage.

In an effort to reduce the occurrence of thrombi formation within the left atrial appendage and prevent thrombi from entering the blood stream from within the left atrial appendage, it may be desirable to develop medical devices and/or occlusive implants that close off the left atrial appendage from the heart and/or circulatory system, thereby lowering the risk of stroke due to thromboembolic material entering the blood stream from the left atrial appendage. Example medical devices and/or occlusive implants that close off the left atrial appendage are disclosed herein.

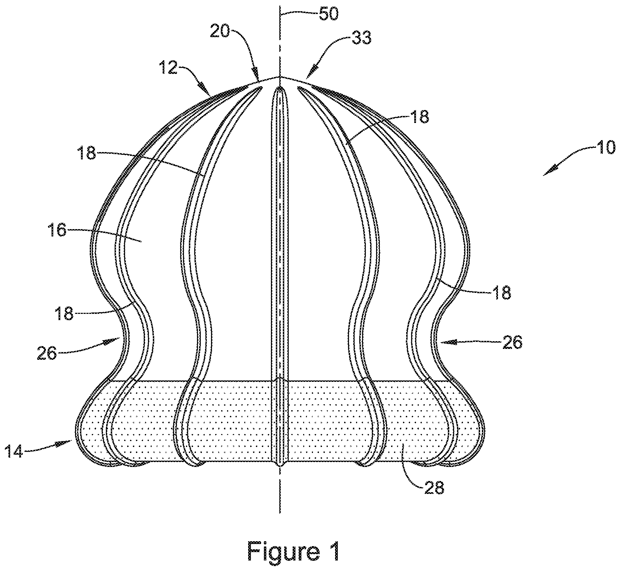

FIG. 1 illustrates an example occlusive implant 10. The occlusive implant 10 may include a first end region 12 and a second end region 14. As will be discussed in greater detail herein, the first end region 12 may include the portion of the occlusive implant 10 which extends farthest into a left atrial appendage, while the second end region 14 may include the portion of the occlusive implant 10 which is positioned closer to an opening of the left atrial appendage.

The occlusive implant 10 may include an expandable member 16. The expandable member 16 may also be referred to as an expandable balloon 16. The expandable member 16 may be formed from a highly compliant material which permits the expandable member 16 to expand from a first unexpanded (e.g., deflated, collapsed, delivery) configuration to a second expanded (e.g., inflated, delivered) configuration with an inflation material or inflation media. In some examples, the expandable balloon 16 may be inflated to pressures from about 27.6 kPa (4 pounds per square inch (psi)) to about 1380 kPa (200 psi). It can be appreciated that the outer diameter of the implant 10 may be larger in the expanded configuration versus the unexpanded configuration. Example materials used for the inflation material may be hydrogel beads (or other semi-solid materials), thermoreversible copolymer, saline, etc.

In some examples, the inflatable member 16 may be constructed from silicone or a low-durometer polymer, however, other materials are contemplated. Additionally, the expandable member 16 may be impermeable to blood and/or other fluids, such as water. In some embodiments, the expandable member 16 may include a woven, braided and/or knitted material, a fiber, a sheet-like material, a metallic or polymeric mesh, or other suitable construction. Further, in some embodiments, the expandable member 16 may prevent thrombi (e.g., blood clots, etc.) originating in the left atrial appendage from passing through the occlusive device 10 and into the blood stream. In some embodiments, the occlusive device 10 may promote endothelial growth after implantation, thereby effectively removing the left atrial appendage from the patient's circulatory system. Some suitable, but non-limiting, examples of materials for the occlusive member 10 are discussed below.

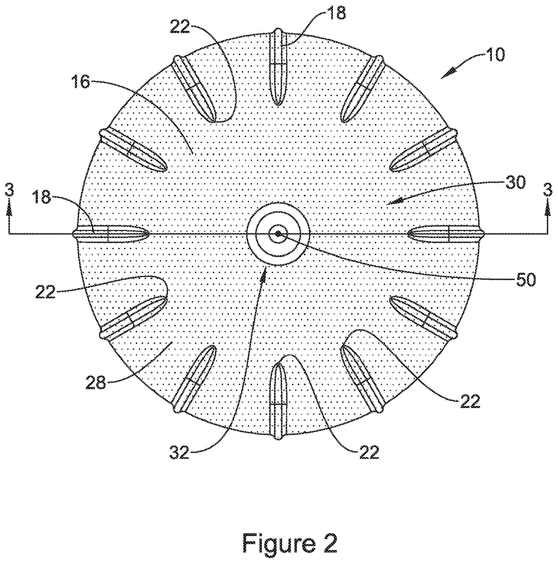

FIG. 1 further illustrates that occlusive member 10 may include one or more spine members 18 extending along the longitudinal axis 50 of the expandable member 16 from the second end region 14 to the first end region 12. In some examples described herein, the spine members 18 may be described as positioning members 18. Each of the spine members 18 may include a first end 20 and a second end 22 (the second end 22 is shown in FIG. 2). FIG. 1 further illustrates that each of the individual spine members 18 may be spaced apart from adjacent spine members 18. In other words, the spacing between adjacent spine members 18 may be substantially uniform around the circumference of the expandable member 16. In some examples, the spine members 18 may include one or more materials which are stiffer, higher durometer materials than the material utilized to construct the expandable member 16. Some suitable, but non-limiting, examples of materials for the spine members 18 are discussed below.

Further, it is contemplated that in some instances the spacing between spine members 18 may not be uniform. In some examples, the spacing between adjacent spine members 18 may be variable (e.g., non-uniformly spaced) around the circumference of the expandable member 16. Additionally, it is contemplated that the spine member 18 may form a framework in which the spine members 18 are connected to one another via a series of laterally extending members. A variety of different geometries for example frameworks are contemplated.

As illustrated in FIG. 1, the first end region 12 of the expandable member 16 may extend radially inward to form an apex region 33. Additionally, as shown in FIG. 1, each of the first end portions 20 of each of the spine members 18 may extend inward along the longitudinal axis 50 toward the apex region 33 of the expandable member 16.

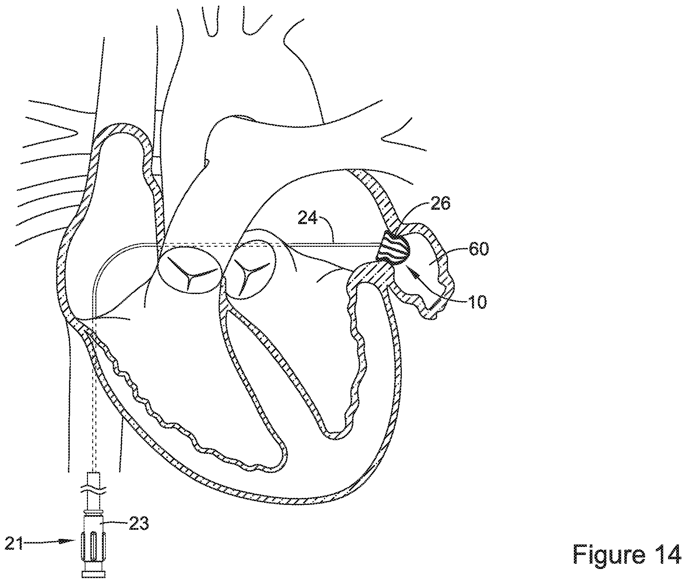

Additionally, FIG. 1 illustrates that the occlusive member 10 may include a "nesting region" 26. The nesting region 26 may define a portion of the occlusive member 10 which is configured to nest within an opening at the ostium of the left atrial appendage (as will be illustrated and described further in FIG. 14). The nesting region 26 may include a portion of the occlusive member 10 which extends radially inward toward the longitudinal axis 50 of the occlusive member 10. Further, the inward curve which defines the nesting region 26 may extend circumferentially around the occlusive member 10. In other words, the inward curvature of the nesting region 26 may resemble a channel or groove which extends around the circumference of the occlusive member 10.

FIG. 1 further illustrates that the second end region 14 of the occlusive member 10 may include a coating 28. The coating 28 may extend around the circumference of the occlusive member 10 (including both the expandable member 16 and the spine members 18). In some examples, the coating 28 may promote cellular growth along the surface thereof. For example, the coating 28 may include elements which promote endothelial growth along the surface thereof. For example, the endothelial growth elements may accelerate the ability for endothelial cellular tissue to form a seal across an opening of the left atrial appendage. In other examples, the coating 28 may include a polymer mesh (e.g., PET mesh), a woven, braided and/or knitted material, a fiber, a sheet-like material, a metallic or polymeric mesh, or other similar materials which may be coupled to the outer surface of the expandable member 16.

FIG. 2 illustrates a bottom view of the occlusive device described in FIG. 1. FIG. 2 illustrates that the occlusive device may include a bottom surface 30. As discussed above, the second end regions 22 of the spine members 18 may "wrap" along (e.g., around) the second end region 14 (shown in FIG. 1) and terminate along the bottom surface 30.

FIG. 2 further shows twelve spine members 18 positioned circumferentially around the longitudinal axis 50 of the occlusive device 10. However, while FIG. 2 illustrates twelve spine members 18 positioned around the longitudinal axis 50 of the occlusive device 10, it is contemplated that greater than or less than twelve spine members 18 may be utilized for any example occlusive devices 10 contemplated herein. For example, occlusive device 10 may include 1, 2, 3, 4, 5, 6, 7, 8, 9, 10, 11, 12, 13, 14, 15, 16 or more spine members 18 positioned along the occlusive device 10.

As will be described in greater detail herein, FIG. 2 further illustrates a valve member 32 positioned in a central region of the bottom surface 30 of the occlusive member 10. The valve 32 may be utilized as an access aperture to insert a secondary medical device (not shown). The secondary medical device may be utilized to inject a fluid material into the expandable member 16. FIG. 2 further illustrates that the coating 28 may be positioned along the bottom surface 30 of the occlusive device 10. The coating 28 may cover all or a portion of the bottom surface 30 of the occlusive device 10.

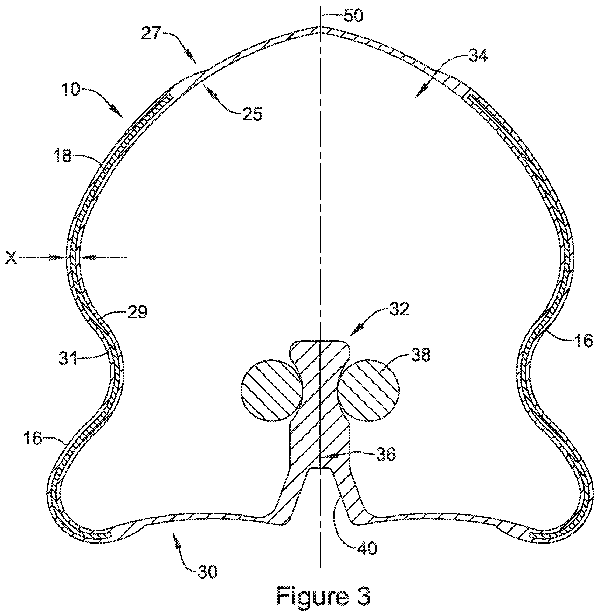

FIG. 3 shows a cross-sectional view along line 3-3 of FIG. 2. FIG. 3 illustrates that the expandable member 16 may include an inner surface 25 and outer surface 27. Additionally, FIG. 3 shows that the expandable member 16 may include a wall thickness "X" defined as the width of the wall between the inner surface 25 and outer surface 27 of the expandable member 16.

FIG. 3 further illustrates that the spine members 18 may be positioned within the wall of the expandable member 16. FIG. 3 illustrates that each of the spine members 18 may include an inwardly-facing surface 29 and an outwardly-facing surface 31. The inner surface 29 of each of the spine members 18 may be positioned radially outward of the inner surface 25 of the expandable member 16. Further, the outer surface 31 of each of the spine members 18 may be positioned radially inward of the outer surface 27 of the occlusive member 10. In other words, each of the spine members 18 be embedded (e.g., encased, surrounded, etc.) within the wall of the expandable member 16. However, this is not intended to be limiting. Rather, it can be appreciated that in some examples, a portion of one or more of the spine members 18 may extend radially away from the outer surface 27 of the expandable member 16. For example, in some instances a portion of the outer surface 31 of one or more of the spine members 18 may be free from the expandable member 16.

FIG. 3 further illustrates that the expandable member 16 may include an inner cavity 34. Inner cavity 34 may be described as a chamber in which an inflation media (for example, but not limited to, hydrogel beads, semi-solid materials, saline or other suitable liquids, gases, etc.) may be injected (via valve 32, for example) in order to expand the expandable member 16. As will be described in greater detail herein, as an inflation media is inserted into the expandable member 16, the inner cavity 34 may expand, thereby permitting the expandable member 16 to seal against the tissue walls defining an opening in the left atrial appendage.

As stated above, inflation of the inner cavity 34 may be accomplished by inserting inflation media through the valve 32. As shown in FIG. 3, the valve 32 may be formed from the same material that forms the wall of the expandable member 16. In other words, the valve 32 may be an extension of the wall of the expandable member 16. Additionally, as illustrated in FIG. 3, the valve 32 may be positioned within the inner cavity 34. For example, FIG. 3 illustrates that the valve 32 may extend (e.g., project) into the inner cavity 34 from the bottom surface 30.

The valve 32 may include an inflation lumen 36 which may be designed to allow a secondary medical device to be inserted therethrough. As shown in FIG. 3, the inflation lumen 36 may be aligned with the longitudinal axis 50 of the occlusive member 10. FIG. 3 shows the inflation lumen 36 in a closed configuration such that it would prevent inflation media (not shown in FIG. 3) from passing back through the valve 32. As shown in FIG. 3, in some examples the valve 32 may be maintained in a closed configuration via a torus-shaped mechanical gasket 38. For simplicity purposes, the gasket 38 may be referred to as an "O-ring" in the remaining discussion. It is contemplated that other sealing mechanisms, such as, but not limited to, one way valves, may also be used to allow for inflation while retaining the inflation media within the cavity. Some illustrative sealing mechanisms are described in commonly assigned U.S. Patent Application Number 62/607,053 filed on December 18, 2017 and titled "OCCLUSIVE DEVICE WITH EXPANDABLE MEMBER".

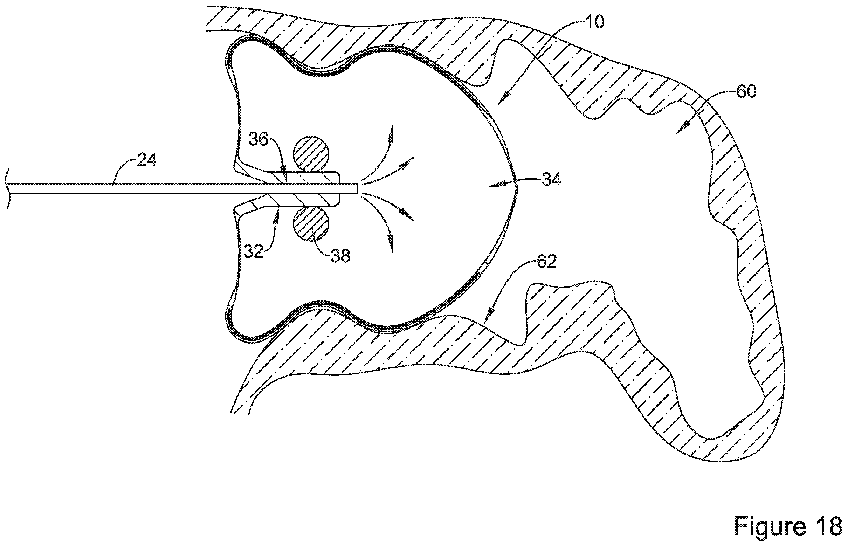

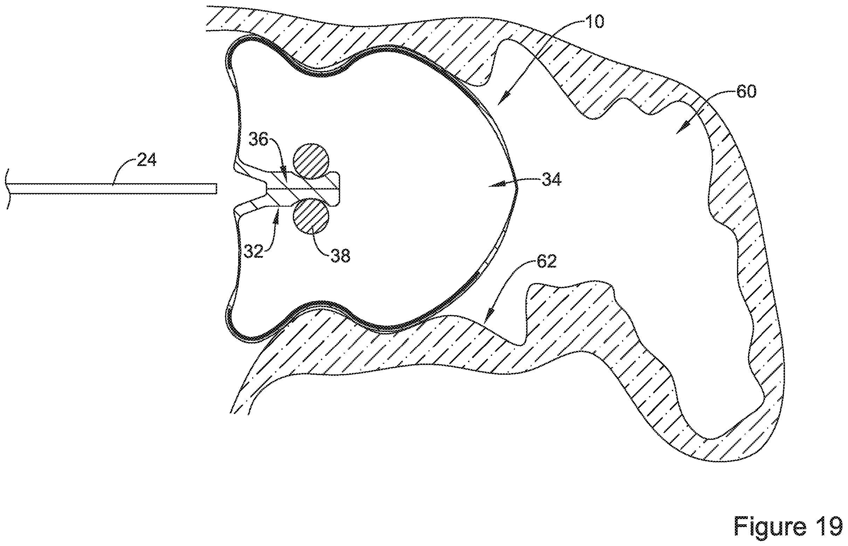

It can be appreciated that the O-ring 38 may be formed from a material (e.g., rubber, elastomer, etc.) which permits it to compress radially inwardly. As shown in FIG. 3, the O-ring 38 may be positioned around the valve 32 such that the O-ring 38 compresses the lumen 36 of valve 32 shut. However, the O-ring 38 must also permit the lumen 36 to open enough for a secondary medical device to be inserted therethrough (for inflation of the expandable member 16 as described above). Therefore, in some examples the O-ring 38 may designed to stretch and allow an inflation device access to the inner chamber 34 while also exerting sufficient radially inward force to maintain the lumen 36 in a closed configuration once the inner chamber 34 has been inflated and after the inflation device (not shown in FIG. 3) is removed from the lumen 36 (inflation of the chamber 34 will be discussed with respect to FIG. 18 and FIG. 19).

As will be discussed in greater detail below, the occlusive member 10 may be coupled to a delivery system in a variety of ways. Further, a component of the delivery system may also function as a secondary medical device utilized to inflate the expandable member 16. FIG. 3 illustrates an attachment region 40 which may be utilized to attach the delivery system to the occlusive member 10. Attachment region 40 may be include a variety of features which permit attachment to a delivery system. For example, attachment region 40 may include threads which mate with a threaded region on a delivery catheter (not shown in FIG. 3). In other examples, the attachment region 40 may be designed such that it forms a "press-fit" with a distal end region of a delivery catheter. Other methods of attaching the occlusive device 10 to the delivery catheter may include a ratcheting mechanism, break-away mechanisms, detent lock, spring lock, single-piece coupling, two-piece coupling, or combinations thereof.

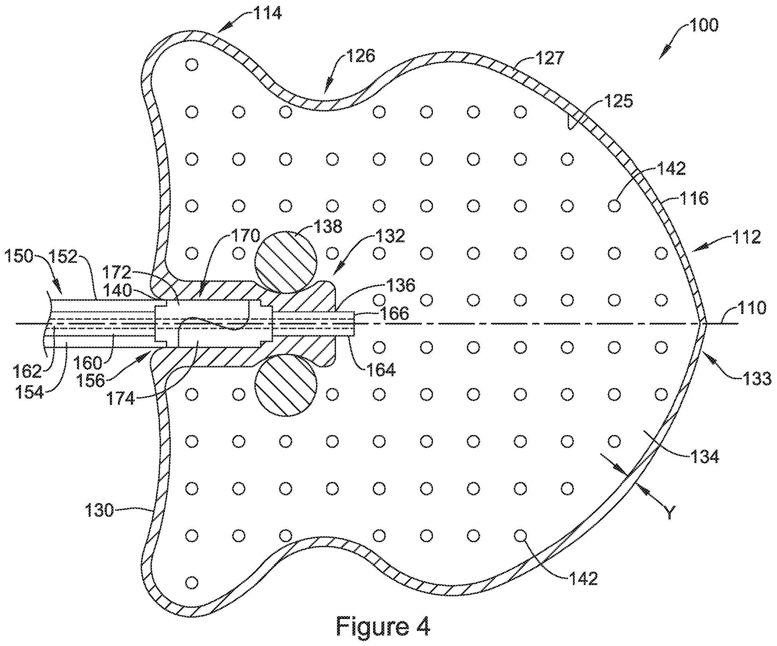

FIG. 4 illustrates a partial cross-sectional view of another example occlusive device 100 in a first inflation state and a side view of a delivery and/or inflation system 150 coupled thereto. The occlusive device 100 may be similar in form and function as the occlusive device 10. For example, the occlusive device 100 may include an expandable member 116 extending along a longitudinal axis 110 from a second end region 114 to a first end region 112. The first end region 112 may include the portion of the occlusive implant 100 which extends farthest into a left atrial appendage, while the second end region 114 may include the portion of the occlusive implant 100 which is positioned closer to an opening of the left atrial appendage. As illustrated in FIG. 4, the first end region 112 of the expandable member 116 may extend radially inward to form an apex region 133. The expandable member 116 may be formed from a highly compliant material which permits the expandable member 116 to expand from a first unexpanded (e.g., deflated, collapsed, delivery) configuration to a second expanded (e.g., inflated, delivered) configuration with an inflation material or inflation media.

While not explicitly shown, the expandable member 116 may include one or more spine members coupled thereto, although this is not required. The expandable member 116 may further include a coating (not explicitly shown). The coating may extend around the circumference of the occlusive member 100. In some examples, the coating may promote cellular growth along the surface thereof. For example, the coating may include elements which promote endothelial growth along the surface thereof. For example, the endothelial growth elements may accelerate the ability for endothelial cellular tissue to form a seal across an opening of the left atrial appendage. In other examples, the coating may include a polymer mesh (e.g., PET mesh), a woven, braided and/or knitted material, a fiber, a sheet-like material, a metallic or polymeric mesh, or other similar materials which may be coupled to the outer surface of the expandable member 116.

Additionally, FIG. 4 illustrates that the occlusive member 100 may include a "nesting region" 126. The nesting region 126 may define a portion of the occlusive member 100 which is configured to nest within an opening of the left atrial appendage. The nesting region 126 may include a portion of the occlusive member 100 which extends radially inward toward the longitudinal axis 110 of the occlusive member 100. Further, the inward curve which defines the nesting region 126 may extend circumferentially around the occlusive member 100. In other words, the inward curvature of the nesting region 126 may resemble a channel or groove which extends around the circumference of the occlusive member 100.

Additionally, the occlusive member 100 may include a valve 132 positioned in central region of a bottom surface 130 of the occlusive member 100. The valve 132 illustrated in FIG. 4 may function in a similar manner as the valve 32 described above. The valve 132 may be utilized as an access aperture to insert a secondary medical device, such as, but not limited to, the delivery and/or inflation system 150. The delivery and/or inflation system 150 may be utilized to deliver occlusive member 100 to the LAA and/or to inject a fluid material into the expandable member 116.

FIG. 4 illustrates that the expandable member 116 may include an inner surface 125 and outer surface 127. Additionally, FIG. 4 shows that the expandable member 116 may include a wall thickness "Y" defined as the width of the wall between the inner surface 125 and outer surface 127 of the expandable member 116.

FIG. 4 further illustrates that the expandable member 116 may include an inner cavity 134. The inner cavity 134 may be described as a chamber in which an inflation media (such as, but not limited to, hydrogel beads, semi-solid materials, saline or other suitable liquids, gases, etc.) may be injected (via valve 132, for example) in order to expand the expandable member 116. As an inflation media is inserted into the expandable member 116, the inner cavity 134 may expand, thereby permitting the expandable member 116 to seal against the tissue walls defining an opening in the left atrial appendage.

Inflation of the inner cavity 134 may be accomplished by inserting inflation media through the valve 132. As shown in FIG. 4, the valve 132 may be formed from the same material that forms the wall of the expandable member 116. In other words, the valve 132 may be an extension of the wall of the expandable member 116. Additionally, the valve 132 may be positioned within the inner cavity 134. For example, FIG. 4 illustrates that the valve 132 may extend (e.g., project) into the inner cavity 134 from the bottom surface 130.

The valve 132 may include an inflation lumen 136 which may be designed allow the delivery and/or inflation system 150 to be inserted therethrough. The delivery and/or inflation system 150 may include an elongate shaft 152 having a lumen 154 extending from a proximal end (not explicitly shown) of the elongate shaft 152 to a distal end 156 of the elongate shaft 152. In some embodiments, the elongate shaft 152 may be a catheter, a hypotube, or other similar tubular structure. In some embodiments, at least a portion of the elongate shaft 152 may include micromachining, a plurality of cuts or weakened areas, some degree of material removal, etc. to provide increased flexibility along a length of the elongate shaft 152 for navigating tortuous vasculature. Some suitable but non-limiting materials for the elongate shaft 152, for example metallic materials, polymer materials, composite materials, etc., are described below.

The delivery and/or inflation system 150 may include a securement member 160 slidably disposed within the lumen 154 of the elongate shaft 152. In some embodiments, the securement member 160 may be a tubular structure, such as but not limited to an elongate shaft, a catheter, a hypotube, or other similar tubular structure. A lumen 162 may extend from a proximal end (not explicitly shown) of the securement member 160 to a distal end 164 of the securement member 160. A distal opening 166 may be positioned at the distal end 164 of the securement member 160.
The occlusive member 100 may be disposed proximate the distal end 156 of the elongate shaft 152. The securement member 160 may be axially slidable between an interlocked position and a released position. The securement member 160 may be configured to releasably attach the occlusive member 100 to the distal end 156 of the elongate shaft 152. In some embodiments, the securement member 160 may be alternately and/or interchangeably referred to as a pull wire, an actuation wire, and/or a locking wire. The securement member 160 may generally be a solid wire or shaft, but may also be tubular in some embodiments. Some suitable but non-limiting materials for the securement member 160, for example metallic materials, polymer materials, composite materials, etc., are described below.

A release mechanism 170 may releasably attach the occlusive member 100 to the distal end 156 of the elongate shaft 152. In some embodiments, the elongate shaft 152 may include a first portion 172 of the release mechanism 170 fixedly attached to the distal end 156 of the elongate shaft 152 and the occlusive member 100 may include a second portion 174 of the release mechanism 170 fixedly attached the occlusive member 100. In some embodiments, the second portion 174 of the release mechanism 170 may be embedded (e.g., encased, surrounded, etc.) within the wall of the valve 132. However, the second portion 174 of the release mechanism 170 may be coupled with the occlusive member 100 using any method desired, including, but not limited to, adhesives, heat melting, overmolding, etc.

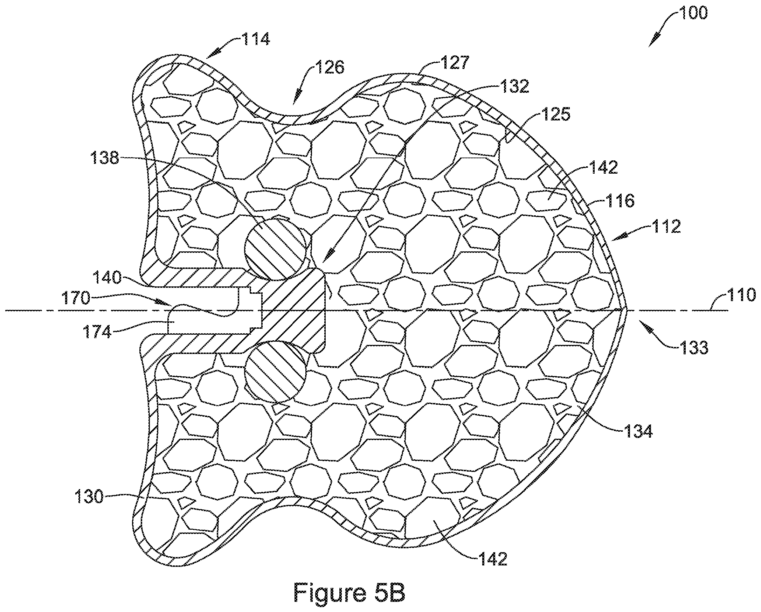

A distal end of the securement member 160 may slidably engage with the first portion 172 of the release mechanism 170 and the second portion 174 of the release mechanism 170 in the interlocked position, as shown in FIG. 4. The securement member 160 interlocks the first portion 172 of the release mechanism 170 with the second portion 174 of the release mechanism 170. The securement member 160 is proximally retracted or translated in a proximal direction relative to the elongate shaft 152 toward a released position to release the second portion 174 of the release mechanism 170 and/or the occlusive member 100 from the first portion 172 of the release mechanism 170 and/or the elongate shaft 152. It is contemplated that the release mechanism 170 may remain in an interlocked configuration until the securement member 160 has been proximally actuated by a length equal to or greater than the length of the release mechanism 170. For example, proximal actuation of the securement member 160 by a length less than a length of the release mechanism 170 may not be sufficient to release the occlusive member 100. In at least some embodiments, the securement member 160 may be slidably disposed within the lumen 154 extending through the elongate shaft 152, a first axial lumen extending through the first portion 172 of the release mechanism 170, and a second axial lumen extending through the second portion 174 of the release mechanism 170. It is contemplated that the release of the occlusive member 100 may be reversed at any axial location of the securement member 160 the interlocked configuration and a fully released configuration (FIG. 5B).

As described above, a component of the delivery system 150 may also function as a secondary medical device utilized to inflate the expandable member 116, although this is not required. FIG. 4 illustrates an attachment region 140 which may be utilized to attach the delivery system 150 to the occlusive member 100. The attachment region 140 may be include a variety of features in addition, or alternatively to the release mechanism 170, which permit attachment to a delivery system. For example, attachment region 140 may include threads which mate with a threaded region on a delivery catheter (not shown in FIG. 4). In other examples, the attachment region 140 may be designed such that it forms a "press-fit" with a distal end region of a delivery catheter. Other methods of attaching the occlusive device 100 to the delivery catheter may include a ratcheting mechanism, break-away mechanisms, detent lock, spring lock, single-piece coupling, two-piece coupling, or combinations thereof.

A proximal end (not explicitly shown) of the lumen 162 of the securement member 160 may be coupled to a hub member (not explicitly shown). An inflation media 142 may be passed through the lumen 162 of the securement member 160 and into the cavity 134 of the expandable member 116. In some embodiments, the inflation media 142 may be hydrogel beads, as shown in FIG. 4. Hydrogel beads 142 may swell upon exposure to aqueous materials. For example, hydrogel beads 142 may absorb aqueous fluids causing the beads to increase in size. The expandable hydrogel may be a semi-solid. The hydrogel beads 142 may be injected, or otherwise transferred, into the cavity 134 of the expandable member 116 in a first stage of expansion of the expandable member 116. This may cause the expandable member 116 to be partially expanded in a first stage. In some instances, the expandable member 116 may be loaded, or partially loaded with hydrogel beads 142 prior to introduction into the body.

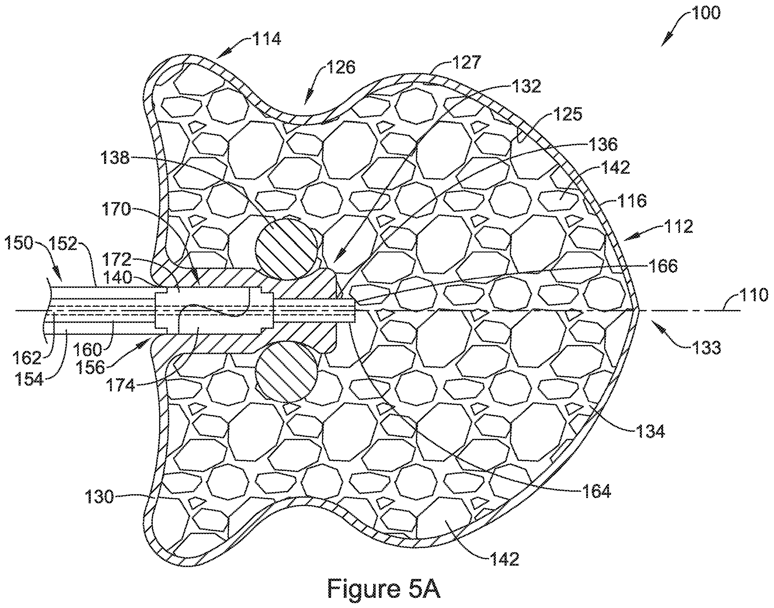

Once the expandable member 116 has been located in the desired position within the LAA, saline, or other aqueous fluid, may be injected through the lumen 162 of the securement member 160 and into the cavity 134 of the expandable member 116 in a second stage of expansion of the expandable member 116. FIG. 5A illustrates a cross-sectional view of the occlusive device 100 in a second expansion state and a side view of a delivery and/or inflation system 150 coupled thereto. The hydrogel beads 142 may absorb the saline causing the hydrogel beads 142 to swell or increase in size, as shown in FIG. 5A. Saline may be injected until the expandable member 116 has reached a desired size. It is contemplated that once the saline has been injected, the inflation media 142 may not be removed and thus the occlusive member 100 may not be repositioned or removable. While the inflation media 142 is described as a hydrogel bead, it is contemplated that other media including, but not limited to, curable media, catalyst activated media, expandable media, (e.g., expanding foams), etc., may be used as desired.

FIG. 5B illustrates a cross-sectional view of the occlusive device 100 in the second expansion state with the delivery and/or inflation system 150 removed. The inflation lumen 136 may be aligned with the longitudinal axis 110 of the occlusive member 100. FIG. 5B shows the inflation lumen 136 in a closed configuration such that it would prevent inflation media 142 from passing back through the valve 132. As shown in FIGS. 4, 5A, and 5B, in some examples the valve 132 may be maintained in a closed configuration via a torus-shaped mechanical gasket 138. For simplicity purposes, the gasket 138 may be referred to as an "O-ring" in the remaining discussion. It is contemplated that other sealing mechanisms, such as, but not limited to, one way valves, may also be used to allow for inflation while retaining the inflation media within the cavity. In some examples, the valve 132 may be a resealable, or punch-through style valve.

It can be appreciated that the O-ring 138 may be formed from a material (e.g., rubber, elastomer, etc.) which permits it to compress radially inwardly. As shown in FIG. 5B, the O-ring 138 may be positioned around the valve 132 such that the O-ring 138 compresses the lumen 136 of valve 132 shut. However, the O-ring 138 must also permit the lumen 136 to open enough for the delivery and/or inflation system 150 to be inserted therethrough (for inflation of the expandable member 116 as described above), as shown in FIGS. 4 and 5A. Therefore, in some examples the O-ring 138 may designed to stretch and allow an inflation device access to the inner chamber 134 while also exerting sufficient radially inward force to maintain the lumen 136 in a closed configuration once the inner chamber 134 has been inflated and after the inflation device 150 is removed from the lumen 136.

In some embodiments, it may be desirable to allow an occlusive member to be repositionable and/or movable. It is contemplated that an inflation media may be used which allows the inflation to the occlusive member to be reversible while allowing for a solid or semi-solid inflation media in the implanted configuration. Such an inflation media may be a thermoreversible copolymer. Thermally reversible or thermoreversible copolymers may have a first state (e.g., a liquid or fluid) at a first temperature and a second state (e.g., a gel) at a second temperature different from the first temperature. For example, the polyethylene glycol-poly(lactic-co-glycolic acid)-polyethylene glycol (PEG-PLGA-PEG) or polyethylene glycol-polycaprolactone-polyethylene glycol (PEG-PCL-PEG) families of copolymers may be a liquid at room temperature (e.g., about 20 to about 25 °C) and form a gel at physiologic temperatures (e.g., about 36 to about 37.5 °C). The thermally reversible inflation media may transition from a liquid to a gel at a transition temperature between room temperature and physiologic temperatures. This may allow a thermally reversible inflation media to gel and create a custom mold fit within the LAA. It is further contemplated that if the position of the occlusive member is not ideal, the internationalist may flush the occlusive implant with a fluid (e.g., saline) at a temperature less than the transition temperature to liquefy the inflation media. The inflation medialsaline solution may then be aspirated. The occlusive member may then be repositioned and re-inflated in the correct position. This inflation media may enable a one size fits all device (e.g., a single device having a size customized to an individual patient) by providing conformability, sealing across a range of LAA sizes in combination with a balloon technology. It may also provide the benefits of reversibility of the inflation media for repositioning, resizing, and recapturing.

Due to the gelling nature of the thermoreversible inflation media, it may be desirable to cool the inflation media as it is delivered to the occlusive member to prevent gelling within the inflation lumen. In some instances, gelling within the lumen of the inflation system may be limited or prevented by flushing the inflation lumen with a cool (e.g., having a temperature less than the transition temperature of the thermoreversible inflation media) fluid (e.g., saline). The flushing fluid may then become integrated into (e.g., absorbed into) or with (e.g., forming a suspension or solution) the thermoreversible inflation media.

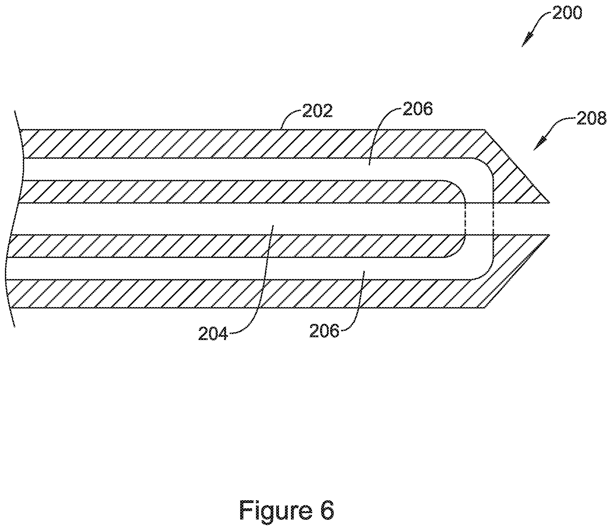

FIG.6 illustrates a cross-sectional view of an inflation system 200 that may be used with a thermoreversible inflation media and the occlusive members described herein. The inflation system 200 may include an elongate shaft 202 having an inflation lumen 204 extending from a proximal end (not explicitly shown) of the elongate shaft 202 to a distal end 208 of the elongate shaft 202. The inflation lumen 204 may be configured to transfer an inflation media from a source outside the body to a cavity of the occlusion member. In some embodiments, the elongate shaft 202 may be a catheter, a hypotube, or other similar tubular structure. In some embodiments, at least a portion of the elongate shaft 202 may include micromachining, a plurality of cuts or weakened areas, some degree of material removal, etc. to provide increased flexibility along a length of the elongate shaft 202 for navigating tortuous vasculature. Some suitable but non-limiting materials for the elongate shaft 202, for example metallic materials, polymer materials, composite materials, etc., are described below.

The elongate shaft 202 may further include a fluid recirculation lumen 206. The recirculation fluid may be a closed loop or non-exposed lumen which allows for fluid to pass alongside the inflation lumen 204 without entering the body or the occlusive member. The recirculation lumen 206 may be configured to receive and circulate a fluid having a temperature less than the transition temperature of the thermoreversible inflation media. The recirculation fluid may keep the elongate shaft 202 at a temperature less than the transition temperature of the thermoreversible inflation media and reduce or prevent gelling of the thermoreversible inflation media until the inflation media reaches the occlusive member. Alternatively, or additionally, the recirculation lumen 206 and/or an auxiliary lumen (not explicitly shown) may include a pop valve (e.g., a valve that opens in response to predetermined pressure) fluidly coupled to the inflation lumen 204. Once the inflation media has been delivered to the occlusion member (and/or at intermittent times during the filling of the occlusion member), the recirculation lumen 206 and/or the auxiliary lumen may be flushed with a fluid at a temperature less than the transition temperature of the thermoreversible inflation media to backflush the inflation lumen 204 and/or remove gelling inflation media from the inflation lumen 204 to prevent or reduce occlusion of the inflation lumen 204.

In addition to allowing for repositioning and/or custom sizing of an expandable occlusive implant, the use of thermoreversible copolymers, such as, but not limited to, PEG-PLGA-PEG or PEG-PCL-PEG families of copolymers may allow for tuning of the inflation media. For example, the properties of the inflation media may be tuned based on the ratio of the polymer blocks in the copolymer. For example, additional polylactic acid (PLA) in the poly(lactic-*co*-glycolic acid structure may increase the Young's modulus. This could be used to tune the polymer to the density of blood, or to approximate the mechanics and compliance of the LAA tissue. Additionally, the components of the media are all either biodegradable (PLGA), or small enough to be cleared by the kidneys (PEG).

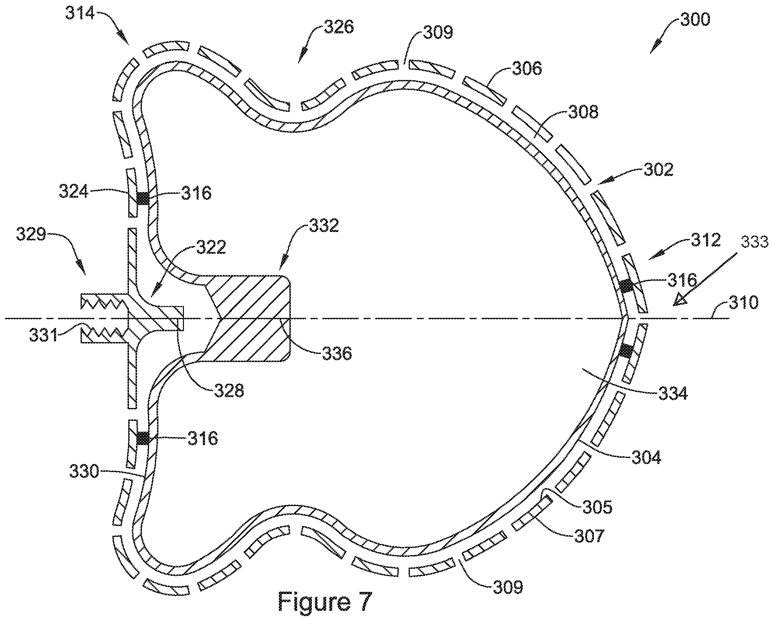

FIG. 7 illustrates a cross-sectional view of another example occlusive device 300. The occlusive device 300 may be similar in form and function as the occlusive devices 10, 100 described herein. The occlusive device 300 may include a co-axial expandable member 302 having a low pressure compliant inner expandable member 304 and an outer perfusion expandable member 306. The inner and outer expandable members 304, 306 may have an outer profile similar in shape. However, the inner expandable member 304 may be sized to be disposed within a cavity 308 of the outer expandable member 306.

The expandable member 302 may extend along a longitudinal axis 310 from a second end region 314 to a first end region 312. The first end region 312 may include the portion of the occlusive implant 300 which extends farthest into a left atrial appendage, while the second end region 314 may include the portion of the occlusive implant 300 which is positioned closer to an opening of the left atrial appendage. As illustrated in FIG. 7, the first end region 312 of the expandable member 302 may extend radially inward to form an apex region 333. The expandable member 302 may be formed from a highly compliant material which permits the expandable member 302 to expand from a first unexpanded (e.g., deflated, collapsed, delivery) configuration to a second expanded (e.g., inflated, delivered) configuration with an inflation material or inflation media.

In some instances, the inner and outer expandable members 304, 306 may be coupled or anchored to one another through one or more septa, walls, or anchors 316 extending between an inner surface of the outer expandable member 306 and an outer surface of the inner expandable member 304. It is contemplated that the inner and outer expandable members 304, 306 may be formed as a unitary structure or separately formed and subsequently coupled, as desired. FIG. 7 illustrates two anchors 316 adjacent to the first end region 312 and two anchors 316 adjacent to the second end region 314. However, this is not required. It is contemplated that the anchors 316 may be spaced or positioned between the inner and outer expandable members 304, 306, as desired. For example, the anchors 316 may be distributed about an outer perimeter and/or a length of the expandable member 302 at uniform intervals or variable intervals. It is further contemplated that the expandable member 302 may include fewer than four or more than four anchors 316, as desired.

While not explicitly shown, the inner expandable member 304 may include one or more spine members coupled thereto, although this is not required. Further, the outer expandable member 306 may further include a coating (not explicitly shown). The coating may extend around the circumference of the occlusive member 300. In some examples, the coating may promote cellular growth along the surface thereof. For example, the coating may include elements which promote endothelial growth along the surface thereof. For example, the endothelial growth elements may accelerate the ability for endothelial cellular tissue to form a seal across an opening of the left atrial appendage. In other examples, the coating may include a polymer mesh (e.g., PET mesh), a woven, braided and/or knitted material, a fiber, a sheet-like material, a metallic or polymeric mesh, or other similar materials which may be coupled to the outer surface of the outer expandable member 306.

Additionally, FIG. 7 illustrates that the occlusive member 300 may include a "nesting region" 326. The nesting region 326 may define a portion of the occlusive member 300 which is configured to nest within an opening of the left atrial appendage. The nesting region 326 may include a portion of the occlusive member 300 which extends radially inward toward the longitudinal axis 310 of the occlusive member 300. Further, the inward curve which defines the nesting region 326 may extend circumferentially around the occlusive member 300. In other words, the inward curvature of the nesting region 326 may resemble a channel or groove which extends around the circumference of the occlusive member 300.

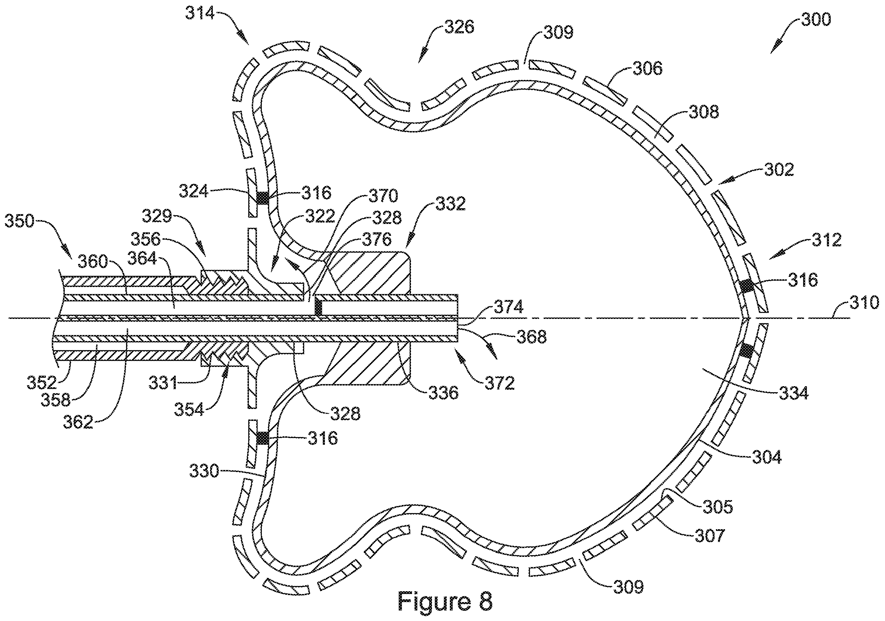

Additionally, the inner expandable member 304 may include a first valve 332 positioned in central region of a bottom surface 330 of the occlusive member 300. In some instances, the valve 332 may be a self-sealing and/or punch-through valve. The valve 332 illustrated in FIG. 7 may function in a similar manner as the valves 32, 132 described above. While not explicitly shown, the valve 332 may include additional structures, such as, but not limited to, o-rings and/or flaps to help maintain the valve 332 in a closed position in the absence of a secondary device extending therethrough. The valve 332 may be utilized as an access aperture to insert a secondary medical device, such as, but not limited to, a delivery and/or inflation system (shown in FIG. 8). The delivery and/or inflation system may be utilized to deliver occlusive member 300 to the LAA and/or to inject a fluid material into the inner expandable member 304 and/or outer expandable member 306.

FIG. 7 further illustrates that the inner expandable member 304 may include an inner cavity 334. The inner cavity 334 may be described as a chamber in which an inflation media (e.g., hydrogel beads, semi-solid materials, thermoreversible copolymers, saline or other suitable liquids, gases, etc.) may be injected (via valve 332, for example) in order to expand the inner expandable member 304. As an inflation media is inserted into the inner expandable member 304 (and/or the outer expandable member 306), the inner cavity 334 (and/or the outer cavity 308) may expand, thereby permitting the occlusive device 300 to seal against the tissue walls defining an opening in the left atrial appendage.

Inflation of the inner cavity 334 may be accomplished by inserting inflation media through the valve 332. As shown in FIG. 7, the valve 332 may be formed from the same material that forms the wall of the inner expandable member 304. In other words, the valve 332 may be an extension of the wall of the inner expandable member 304. Additionally, the valve 332 may be positioned within the inner cavity 334. For example, FIG. 7 illustrates that the valve 332 may extend (e.g., project) into the inner cavity 334 from the bottom surface 330.

The valve 332 may include an inflation lumen 336 which may be designed allow the delivery and/or inflation system to be inserted therethrough. The inflation lumen 336 may be aligned with the longitudinal axis 310 of the occlusive member 300. FIG. 7 shows the inflation lumen 336 in a closed configuration such that it would prevent inflation media (not explicitly shown) from passing back through the valve 332.

Additionally, the outer expandable member 306 may include a second valve 322 positioned in central region of a bottom surface 324 of the occlusive member 300. In some instances, the valve 322 may be a self-sealing and/or punch-through valve. The valve 322 illustrated in FIG. 7 may function in a similar manner as the valves 32, 132, 332 described above. While not explicitly shown, the valve 322 may include additional structures, such as, but not limited to, o-rings and/or flaps to help maintain the valve 332 in a closed position in the absence of a secondary device extending therethrough. The valve 322 may be utilized as an access aperture to insert a secondary medical device, such as, but not limited to, a delivery and/or inflation system (shown in FIG. 8). The delivery and/or inflation system may be utilized to deliver occlusive member 300 to the LAA and/or to inject a fluid material into the inner expandable member 304 and/or outer expandable member 306.

FIG. 7 further illustrates that the outer expandable member 306 may include an inner cavity 308. The inner cavity 308 may be described as a chamber in which an inflation media (e.g., hydrogel beads, semi-solid materials, thermoreversible copolymers, saline or other suitable liquids, gases, etc.) may be injected (via valve 322, for example) in order to expand the outer expandable member 306. As an inflation media is inserted into the outer expandable member 306, the inner cavity 308 may expand, thereby permitting the occlusive device 300 to seal against the tissue walls defining an opening in the left atrial appendage.

In some embodiments, the outer expandable member 306 may include a plurality of (e.g., one or more) perfusion apertures 309 extending from an inner surface 305 to an outer surface 307 of the outer expandable member 306. The apertures 309 may be configured to allow an inflation media to weep or leak from the cavity 308 of the outer expandable member 306 and into the LAA. When a gelling material (such as, but not limited to, a thermoreversible copolymer or a hydrogel material) is used, gelation may occur within the cavity 308, through the apertures 309, and within the internal LAA. This may mechanically fixate the occlusive device 300 to and/or within the LAA. The apertures 309 may extend over an entire surface of the outer expandable member 306 or along portions thereof. For example, in some embodiments, the apertures 309 may be limited to regions of the outer expandable member 306 expected to be in close proximity to a wall or tissue of the LAA.

Inflation of the inner cavity 308 and/or introduction of a perfusion media may be accomplished by inserting inflation media through the valve 322. As shown in FIG. 7, the valve 322 may be formed from the same material that forms the wall of the outer expandable member 306. In other words, the valve 322 may be an extension of the wall of the outer expandable member 306. Additionally, the valve 322 may be positioned interior to the inner cavity 308.

The valve 322 may include an inflation lumen 328 which may be designed allow the delivery and/or inflation system to be inserted therethrough. The inflation lumen 328 may be aligned with the longitudinal axis 310 of the occlusive member 300. FIG. 7 shows the inflation lumen 328 in a closed configuration such that it would prevent inflation media (not explicitly shown) from passing back through the valve 322.

FIG. 7 illustrates an attachment region 329 of the outer expandable member 306 which may be utilized to attach a delivery system (see, for example, FIG. 8) to the occlusive member 300. As shown in FIG. 7, the attachment region 329 may be formed from the same material that forms the wall of the outer expandable member 306, although this is not required. The attachment region 329 may be include a variety of features in addition, or alternatively to a threaded region 331, which permit attachment to a delivery system. In other examples, the attachment region 339 may be designed such that it forms a "press-fit" with a distal end region of a delivery catheter. Other methods of attaching the occlusive device 300 to the delivery catheter may include a ratcheting mechanism, break-away mechanisms, detent lock, spring lock, single-piece coupling, two-piece coupling, or combinations thereof. In some embodiments, the attachment region 329 may extend inwards towards the cavity 308 of the outer expandable member 306.

FIG. 8 is a cross-sectional view of the illustrative occlusive device 300 of FIG. 7 coupled with an illustrative delivery and/or inflation system 350. The delivery and/or inflation system 350 may include an elongate shaft 352 having a lumen 358 extending from a proximal end (not explicitly shown) of the elongate shaft 352 to a distal end 354 of the elongate shaft 352. In some embodiments, the elongate shaft 352 may be a catheter, a hypotube, or other similar tubular structure. In some embodiments, at least a portion of the elongate shaft 352 may include micromachining, a plurality of cuts or weakened areas, some degree of material removal, etc. to provide increased flexibility along a length of the elongate shaft 352 for navigating tortuous vasculature. Some suitable but non-limiting materials for the elongate shaft 352, for example metallic materials, polymer materials, composite materials, etc., are described below. The distal end 354 of the elongate shaft 352 may include a threaded region 356 configured to threadably and releasably engage a threaded region 331 of the attachment region 329. This may allow the delivery and/or inflation system 350 to be releaseably coupled to the occlusive device 300. In other examples, the valve 322 may be designed such that it forms a "press-fit" with a distal end region of a delivery catheter 350. Other methods of attaching the occlusive device 300 to the delivery catheter may include a ratcheting mechanism, break-away mechanisms, detent lock, spring lock, single-piece coupling, two-piece coupling, or combinations thereof.

The delivery and/or inflation system 350 may include an inner elongate shaft 360 disposed within the lumen 358 of the elongate shaft 352. The inner elongate shaft 360 may be a tubular structure, such as but not limited to an elongate shaft, a catheter, a hypotube, or other similar tubular structure. In some embodiments, the inner elongate shaft 360 may be slidably disposed within the lumen 358 of the elongate shaft 352. In other embodiments, the inner elongate shaft 360 may be fixedly coupled within the lumen 358 of the elongate shaft 352. A first lumen 362 may extend from a proximal end (not explicitly shown) of inner elongate shaft 360 to a distal end 372 of the inner elongate shaft 360. A distal opening 374 may be positioned at the distal end 372 of the inner elongate shaft 360. The inner elongate shaft 360 may further include a second lumen 364 extending from a proximal end (not explicitly shown) of inner elongate shaft 360 to an opening 376 positioned proximal to the distal end 372 of the inner elongate shaft 360. While the inner elongate shaft 360 is illustrated as having a side-by side dual lumen arrangement, it is contemplated that the first and second lumens 362, 364 may be provided in a co-axial (or tube within a tube) arrangement, as desired.

A proximal end (not explicitly shown) of the first lumen 362 of the inner elongate shaft 360 may be coupled to a hub member (not explicitly shown). An inflation media 368 may be passed through the first lumen 362 of the inner elongate shaft 360 and into the cavity 334 of the inner expandable member 304.

A proximal end (not explicitly shown) of the second lumen 364 of the inner elongate shaft 360 may be coupled to a hub member (not explicitly shown). An inflation media 370 may be passed through the second lumen 364 of the inner elongate shaft 360 and into the cavity 308 of the outer expandable member 306. As described above, some of the inflation media 370 may be configured to weep from or exit the outer expandable member 306 through one or more apertures 309. In some embodiments, the inflation media 368 may be a thermoreversible copolymer or other media configured to gel or transition to a semi-solid upon delivery, although this is not required. In some instances, the inflation media 368 provided to the cavity 334 of the inner expandable member 304 may be cooled to cool the inflation media 370 provided to the cavity 308 of the outer expandable member 308 to prevent or reduce gelation of the inflation mediate 370 prior to filling the entire cavity 308. It is contemplated that the inflation media 368 may be different from the inflation media 370 used in the cavity 308 of the outer expandable member 306.

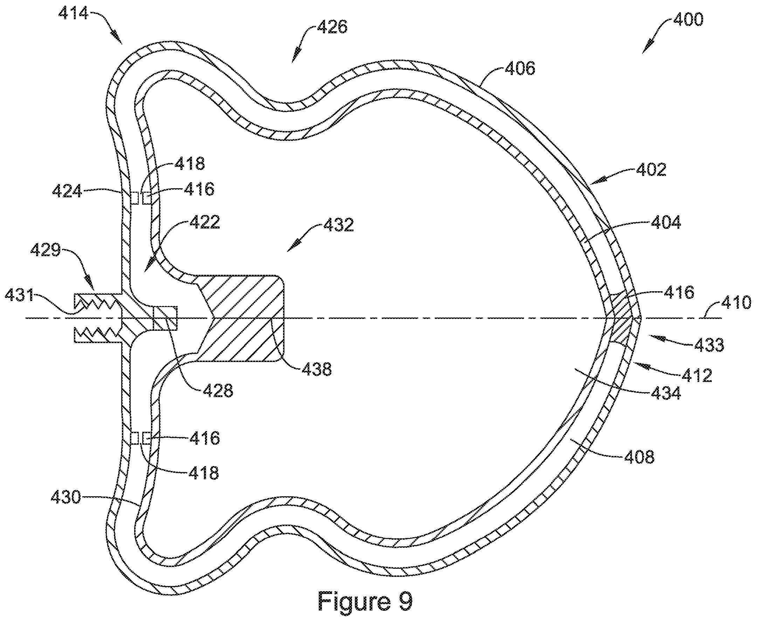

FIG. 9 illustrates a cross-sectional view of another example occlusive device 400. The occlusive device 400 may be similar in form and function as the occlusive devices 10, 100, 300 described herein. The occlusive device 400 may include a co-axial expandable member 402 having an inner expandable member 404 and an expandable outer member 406. The inner and outer expandable members 404, 406 may have an outer profile similar in shape. However, the inner expandable member 404 may be sized to be disposed within a cavity 408 of the outer expandable member 406.

The expandable member 402 may extend along a longitudinal axis 410 from a second end region 414 to a first end region 412. The first end region 412 may include the portion of the occlusive implant 400 which extends farthest into a left atrial appendage, while the second end region 414 may include the portion of the occlusive implant 400 which is positioned closer to an opening of the left atrial appendage. As illustrated in FIG. 9, the first end region 412 of the expandable member 402 may extend radially inward to form an apex region 433. The inner and/or outer expandable members 404, 406 may be formed from a highly compliant material which permits the expandable member 402 to expand from a first unexpanded (e.g., deflated, collapsed, delivery) configuration to a second expanded (e.g., inflated, delivered) configuration with an inflation material or inflation media.

In some instances, the inner and outer expandable members 404, 406 may be coupled or anchored to one another through one or more septa, walls, or anchors 416 extending between an inner surface of the outer expandable member 406 and an outer surface of the inner expandable member 404. It is contemplated that the inner and outer expandable members 404, 406 may be formed as a unitary structure or separately formed and subsequently coupled, as desired. FIG. 7 illustrates one anchor 416 adjacent to the first end region 412 and two anchors 416 adjacent to the second end region 414. However, this is not required. It is contemplated that the anchors 416 may be spaced or positioned between the inner and outer expandable members 404, 406, as desired. For example, the anchors 416 may be distributed about an outer perimeter and/or a length of the expandable member 402 at uniform intervals or variable intervals. It is further contemplated that the expandable member 402 may include fewer than three or more than three anchors 416, as desired. In some embodiments, one or more of the anchors 416 may including openings 418 extending therethrough to facilitate passage of an inflation media.

While not explicitly shown, the inner and/or outer expandable members 404, 406 may include one or more spine members coupled thereto, although this is not required. Further, the outer expandable member 406 may further include a coating (not explicitly shown). The coating may extend around the circumference of the occlusive member 400. In some examples, the coating may promote cellular growth along the surface thereof. For example, the coating may include elements which promote endothelial growth along the surface thereof. For example, the endothelial growth elements may accelerate the ability for endothelial cellular tissue to form a seal across an opening of the left atrial appendage. In other examples, the coating may include a polymer mesh (e.g., PET mesh), a woven, braided and/or knitted material, a fiber, a sheet-like material, a metallic or polymeric mesh, or other similar materials which may be coupled to the outer surface of the outer expandable member 406.

Additionally, FIG. 9 illustrates that the occlusive member 400 may include a "nesting region" 426. The nesting region 426 may define a portion of the occlusive member 400 which is configured to nest within an opening of the left atrial appendage. The nesting region 426 may include a portion of the occlusive member 400 which extends radially inward toward the longitudinal axis 410 of the occlusive member 400. Further, the inward curve which defines the nesting region 426 may extend circumferentially around the occlusive member 400. In other words, the inward curvature of the nesting region 426 may resemble a channel or groove which extends around the circumference of the occlusive member 400.

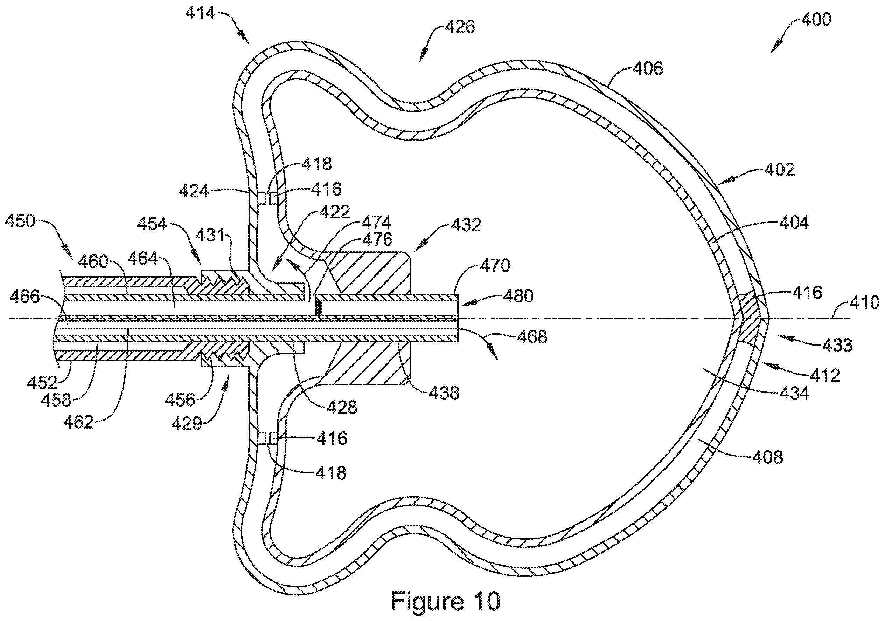

Additionally, the inner expandable member 404 may include a first valve 432 positioned in central region of a bottom surface 430 of the occlusive member 400. The valve 432 illustrated in FIG. 9 may function in a similar manner as the valves 32, 132, 322, 332 described above. In some instances, the valve 432 may be a self-sealing and/or punch-through valve. While not explicitly shown, the valve 432 may include additional structures, such as, but not limited to, o-rings and/or flaps to help maintain the valve 432 in a closed position in the absence of a secondary device extending therethrough. The valve 432 may be utilized as an access aperture to insert a secondary medical device, such as, but not limited to, a delivery and/or inflation system (shown in FIGS. 10-12). The delivery and/or inflation system may be utilized to deliver occlusive member 400 to the LAA and/or to inject a fluid material into the inner expandable member 404 and/or outer expandable member 406.

FIG. 9 further illustrates that the inner expandable member 404 may include an inner cavity 434. The inner cavity 434 may be described as a chamber in which an inflation media (e.g., hydrogel beads, semi-solid materials, thermoreversible copolymers, saline or other suitable liquids, gases, etc.) may be injected (via valve 432, for example) in order to expand the inner expandable member 404. As an inflation media is inserted into the inner expandable member 404, the inner cavity 434 may expand, thereby permitting the occlusive device 400 to seal against the tissue walls defining an opening in the left atrial appendage.

Inflation of the inner cavity 434 may be accomplished by inserting inflation media through the valve 432. As shown in FIG. 9, the valve 432 may be formed from the same material that forms the wall of the inner expandable member 404. In other words, the valve 432 may be an extension of the wall of the inner expandable member 404. Additionally, the valve 432 may be positioned within the inner cavity 434. For example, FIG. 9 illustrates that the valve 432 may extend (e.g., project) into the inner cavity 434 from the bottom surface 430.

The valve 432 may include an inflation lumen 438 which may be designed allow the delivery and/or inflation system to be inserted therethrough. The inflation lumen 438 may be aligned with the longitudinal axis 410 of the occlusive member 400. FIG. 9 shows the inflation lumen 438 in a closed configuration such that it would prevent inflation media (not explicitly shown) from passing back through the valve 432. While not explicitly shown, the valve 432 may include additional structures, such as, but not limited to, o-rings and/or flaps to help maintain the valve 332 in a closed position in the absence of a secondary device extending therethrough.

Additionally, the outer expandable member 406 may include a second valve 422 positioned in central region of a bottom surface 424 of the occlusive member 400. In some instances, the valve 422 may be a self-sealing and/or punch-through valve. The valve 422 illustrated in FIG. 9 may function in a similar manner as the valves 32, 132, 322, 332 described above. While not explicitly shown, the valve 422 may include additional structures, such as, but not limited to, o-rings and/or flaps to help maintain the valve 422 in a closed position in the absence of a secondary device extending therethrough. The valve 422 may be utilized as an access aperture to insert a secondary medical device, such as, but not limited to, a delivery and/or inflation system (shown in FIGS. 10-12). The delivery and/or inflation system may be utilized to deliver occlusive member 400 to the LAA and/or to inject a fluid material into the inner expandable member 404 and/or outer expandable member 406.

FIG. 9 further illustrates that the outer expandable member 406 may include a cavity 408. While the cavity 408 is within the outer expandable member 406, it may be described as an outer cavity 408 with respect to its spatial relationship to the inner expandable member 404. The cavity 408 may be described as a chamber in which an inflation media (e.g., hydrogel beads, semi-solid materials, thermoreversible copolymers, saline or other suitable liquids, gases, etc.) may be injected (via valve 422, for example) in order to expand the outer expandable member 406. As will be described in greater detail herein, as an inflation media is inserted into the outer expandable member 406, the outer cavity 408 may expand, thereby permitting the occlusive device 400 to seal against the tissue walls defining an opening in the left atrial appendage.

Inflation of the outer cavity 408 may be accomplished by inserting inflation media through the valve 422. As shown in FIG. 9, the valve 422 may be formed from the same material that forms the wall of the outer expandable member 406. In other words, the valve 422 may be an extension of the wall of the outer expandable member 406. Additionally, the valve 422 may be positioned interior to the inner cavity 408.

The valve 422 may include an inflation lumen 428 which may be designed allow the delivery and/or inflation system to be inserted therethrough. The inflation lumen 428 may be aligned with the longitudinal axis 410 of the occlusive member 400. FIG. 9 shows the inflation lumen 428 in a closed configuration such that it would prevent inflation media (not explicitly shown) from passing back through the valve 422.

FIG. 9 illustrates an attachment region 429 of the outer expandable member 406 which may be utilized to attach a delivery system (see, for example, FIGS. 10-12) to the occlusive member 400. As shown in FIG. 9, the attachment region 429 may be formed from the same material that forms the wall of the outer expandable member 406, although this is not required. The attachment region 429 may be include a variety of features in addition, or alternatively to a threaded region 431, which permit attachment to a delivery system. In other examples, the attachment region 429 may be designed such that it forms a "press-fit" with a distal end region of a delivery catheter. Other methods of attaching the occlusive device 400 to the delivery catheter may include a ratcheting mechanism, break-away mechanisms, detent lock, spring lock, single-piece coupling, two-piece coupling, or combinations thereof. In some embodiments, the attachment region 429 may extend inwards towards or within the cavity 408 of the outer expandable member 406.

FIG. 10 is a cross-sectional view of the illustrative occlusive device 400 of FIG. 9 coupled with an illustrative delivery and/or inflation system 450. The delivery and/or inflation system 450 may include an elongate shaft 452 having a lumen 458 extending from a proximal end (not explicitly shown) of the elongate shaft 452 to a distal end 454 of the elongate shaft 452. In some embodiments, the elongate shaft 452 may be a catheter, a hypotube, or other similar tubular structure. In some embodiments, at least a portion of the elongate shaft 452 may include micromachining, a plurality of cuts or weakened areas, some degree of material removal, etc. to provide increased flexibility along a length of the elongate shaft 452 for navigating tortuous vasculature. Some suitable but non-limiting materials for the elongate shaft 452, for example metallic materials, polymer materials, composite materials, etc., are described below. The distal end 454 of the elongate shaft 452 may include a threaded region 456 configured to threadably and releasably engage a threaded region 431 of the attachment region 429. This may allow the delivery and/or inflation system 450 to be releaseably coupled to the occlusive device 400. In other examples, the valve 422 may be designed such that it forms a "press-fit" with a distal end region of a delivery catheter 450. Other methods of attaching the occlusive device 400 to the delivery catheter may include a ratcheting mechanism, break-away mechanisms, detent lock, spring lock, single-piece coupling, two-piece coupling, or combinations thereof.

The delivery and/or inflation system 450 may include an inner elongate shaft 460 disposed within the lumen 458 of the elongate shaft 452. The inner elongate shaft 460 may be a tubular structure, such as but not limited to an elongate shaft, a catheter, a hypotube, or other similar tubular structure. In some embodiments, the inner elongate shaft 460 may be slidably disposed within the lumen 458 of the elongate shaft 452. In other embodiments, the inner elongate shaft 460 may be fixedly coupled within the lumen 458 of the elongate shaft 452. A first inflation lumen 462 may extend from a proximal end (not explicitly shown) of the inner elongate shaft 460 to a distal end 470 of the inner elongate shaft 460. A distal opening 480 may be positioned at the distal end 470 of the inner elongate shaft 460. The inner elongate shaft 460 may further include a second inflation lumen 464 extending from a proximal end (not explicitly shown) of inner elongate shaft 460 to an opening 476 positioned proximal to the distal end 470 of the inner elongate shaft 460. A third lumen 466 may extend from a proximal end (not explicitly shown) of the inner elongate shaft 460 to a distal end 470 of the inner elongate shaft 460. The third lumen 466 may be configured to remove an inflation media from the inner cavity 434, although this is not required. While the inner elongate shaft 460 is illustrated as having a side-by side lumen arrangement, it is contemplated that the first, second, and third lumens 462, 464, 466 may be provided in a co-axial (or tube within a tube) arrangement, as desired.

A proximal end (not explicitly shown) of the second inflation lumen 464 of the inner elongate shaft 460 may be coupled to a hub member (not explicitly shown). An inflation media 474 may be passed through the second inflation lumen 464 of the inner elongate shaft 460 and into the cavity 408 of the outer expandable member 406. In some embodiments, the inflation media 474 may be a thermoreversible copolymer or other media configured to gel or transition to a semi-solid upon delivery, although this is not required.

A proximal end (not explicitly shown) of the first inflation lumen 462 of the inner elongate shaft 460 may be coupled to a hub member (not explicitly shown). An inflation media 468 may be passed through the first lumen 462 of the inner elongate shaft 460 and into the cavity 434 of the inner expandable member 404. It is contemplated that the inflation media 468 may be the same as or different from the inflation media used in the cavity 408 of the outer expandable member 406. A proximal end (not explicitly shown) of the third lumen 466 of the inner elongate shaft 460 may be coupled to a hub member or a suction device (not explicitly shown) to remove the inflation material from the cavity 434 of the inner expandable member 404.

In some embodiments, a thermoreversible copolymer inflation media 474 may be injected into the cavity 408 of the outer expandable member 406 and a cooling inflation media or fluid 468 (such as, but not limited to, saline) at a temperature less than the transition temperature of the thermoreversible inflation media 474 may be injected into the cavity 434 of the inner expandable member 404. Cold inflation media 468 may be continuously circulated within the cavity 434 of the inner expandable member 404 (e.g., injected through the first lumen 468 and removed through the third lumen 466) to prevent the thermoreversible copolymer inflation media 474 from gelling prematurely. Once the placement of the occlusion deice 400 has been verified, saline 468 at body temperature may be circulated into the cavity 434 of the inner expandable member 404 to cause the thermoreversible copolymer inflation media 474 in the cavity 408 of the outer expandable member 406 to gel. At any time prior to uncoupling the delivery system 450 from the occlusive device 400, cold saline 468 (at a temperature less than the transition temperature of the thermoreversible inflation media 474) may be recirculated into the cavity 434 of the inner expandable member 404 to soften or liquefy the thermoreversible inflation media 474 in the cavity 408 of the outer expandable member 406. The liquid inflation media 474 may then be removed or partially removed from the outer cavity 408. This may allow the occlusive member 400 to be removed, repositioned, and/or re-formed to the anatomy of the LAA. Further, the inflation media 474, 468 may be removed from both cavities 408, 434 if full recapture is desired and/or necessary.

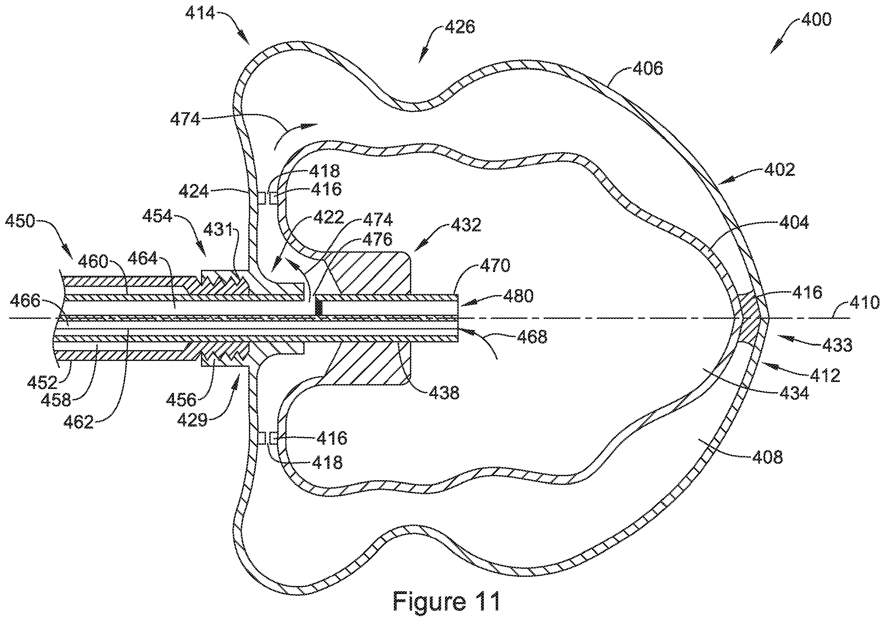

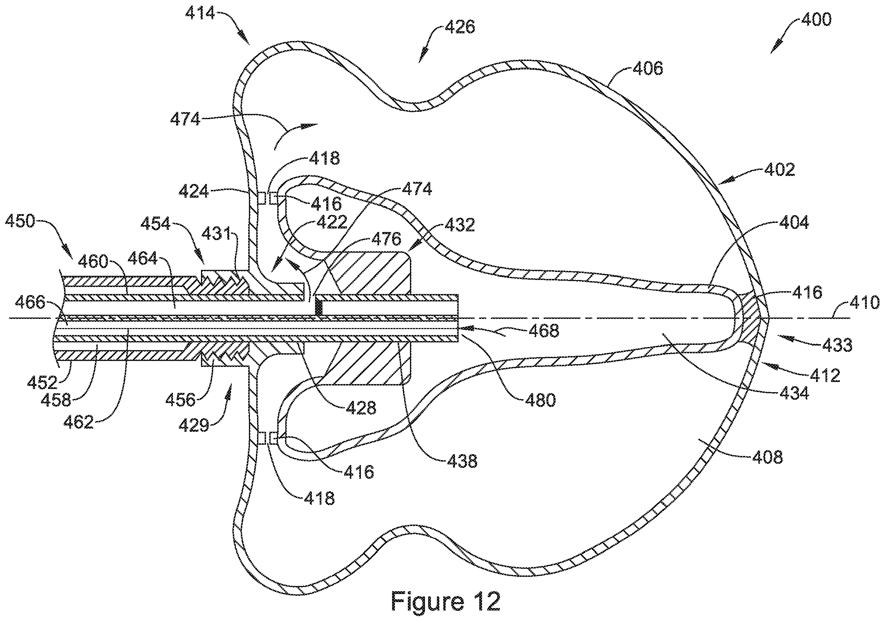

In some embodiments, the cavity 434 of the inner expandable member 404 may remain filled with the saline inflation media 468. In other embodiments, the saline (or other inflation media) 468 may be removed (e.g., through the third lumen 466) as additional thermoreversible inflation media 474 is injected into the cavity 408 of the outer expandable member 406, as shown in FIG. 11 which is another cross-sectional view of the illustrative occlusive device 400 of FIG. 9 coupled with the illustrative delivery and/or inflation system 450. The saline 468 may continue to be removed as additional thermoreversible inflation media 474 is injected into the cavity 408 of the outer expandable member 406, as shown in FIG. 12, until the inner expandable member 404 has collapsed and the occlusive device 400 is filled with thermoreversible inflation media 474. While the inflation media 468 is described as being removed through a third lumen 466, this is not required. In some examples, the first lumen 462 may be used to inject and remove inflation media 468 from the inner cavity 434.

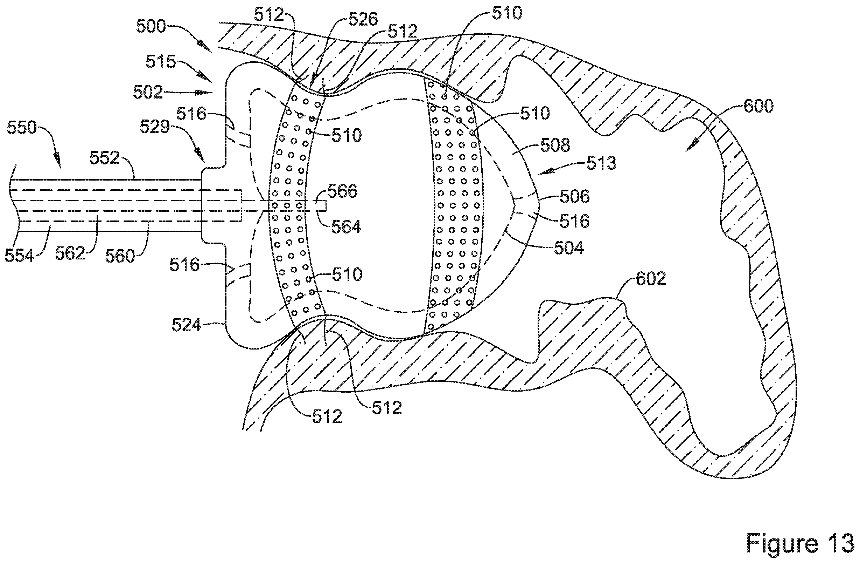

FIG. 13 illustrates a side view of another example occlusive device 500 positioned within an opening at the ostium of a left atrial appendage 600. The occlusive device 500 may be similar in form and function as the occlusive devices 10, 100, 300, 400 described herein. The occlusive device 500 may include a co-axial expandable member 502 having an inner expandable member 504 and an outer expandable member 506. The inner and outer expandable members 504, 506 may have an outer profile similar in shape. However, the inner expandable member 504 may be sized to be disposed within a cavity 508 of the outer expandable member 506. The inner and/or outer expandable members 504, 506 may be formed from a highly compliant material which permits the expandable member 502 to expand from a first unexpanded (e.g., deflated, collapsed, delivery) configuration to a second expanded (e.g., inflated, delivered) configuration with an inflation material or inflation media.

In some instances, the inner and outer expandable members 504, 506 may be coupled or anchored to one another through one or more septa, walls, or anchors 516 extending between an inner surface of the outer expandable member 506 and an outer surface of the inner expandable member 504. It is contemplated that the inner and outer expandable members 504, 506 may be formed as a unitary structure or separately formed and subsequently coupled, as desired. FIG. 13 illustrates one anchor 516 adjacent to a first end region 513 and two anchors 516 adjacent to the second end region 515. However, this is not required. It is contemplated that the anchors 516 may be spaced or positioned between the inner and outer expandable members 504, 506, as desired. For example, the anchors 516 may be distributed about an outer perimeter and/or a length of the expandable member 502 at uniform intervals or variable intervals. It is further contemplated that the expandable member 502 may include fewer than three or more than three anchors 516, as desired.

While not explicitly shown, the inner and/or outer expandable members 504, 506 may include one or more spine members coupled thereto, although this is not required. Further, the outer expandable member 506 may further include a coating (not explicitly shown). The coating may extend around the circumference of the occlusive member 500. In some examples, the coating may promote cellular growth along the surface thereof. For example, the coating may include elements which promote endothelial growth along the surface thereof. For example, the endothelial growth elements may accelerate the ability for endothelial cellular tissue to form a seal across an opening of the left atrial appendage. In other examples, the coating may include a polymer mesh (e.g., PET mesh), a woven, braided and/or knitted material, a fiber, a sheet-like material, a metallic or polymeric mesh, or other similar materials which may be coupled to the outer surface of the outer expandable member 506.

Additionally, FIG. 13 illustrates that the occlusive member 500 may include a "nesting region" 526. The nesting region 526 may define a portion of the occlusive member 500 which is configured to nest within an opening of the left atrial appendage 600. The nesting region 526 may include a portion of the occlusive member 500 which extends radially inward toward the longitudinal axis of the occlusive member 500. Further, the inward curve which defines the nesting region 526 may extend circumferentially around the occlusive member 500. In other words, the inward curvature of the nesting region 526 may resemble a channel or groove which extends around the circumference of the occlusive member 500.

Additionally, the inner expandable member 504 may be similar in form and function the inner expandable members 304, 404 described herein. While not explicitly shown, the expandable member 504 may include a first valve positioned in central region of a bottom surface of the occlusive member 500. The valve may function in a similar manner as the valves 32, 132, 322, 332, 422, 432 described above. The valve may be utilized as an access aperture to insert a secondary medical device, such as, but not limited to, a delivery and/or inflation system (not explicitly shown). The delivery and/or inflation system may be utilized to deliver occlusive member 500 to the LAA and/or to inject a fluid material into the inner expandable member 504 and/or outer expandable member 506.

While not explicitly shown, the inner expandable member 504 may include an inner cavity similar in form and function the inner cavities 334, 434 described herein. The inner cavity may be described as a chamber in which an inflation media (e.g., hydrogel beads, semi-solid materials, thermoreversible copolymers, saline or other suitable liquids, gases, etc.) may be injected (via valve 432, for example) in order to expand the inner expandable member 504. As an inflation media is inserted into the inner expandable member 504, the inner cavity may expand, thereby permitting the occlusive device 500 to seal against the tissue walls defining an opening in the left atrial appendage. Inflation of the inner cavity may be accomplished by inserting inflation media through the valve.

Additionally, the outer expandable member 506 may include a second valve (not explicitly shown) positioned in central region of a bottom surface of the occlusive member 500. The valve may function in a similar manner as the valves 32, 132, 322, 332, 422, 432 described above. The valve may be utilized as an access aperture to insert a secondary medical device, such as, but not limited to, a delivery and/or inflation system 550. The delivery and/or inflation system 550 may be utilized to deliver occlusive member 500 to the LAA and/or to inject a fluid material into the inner expandable member 504 and/or outer expandable member 506.

FIG. 13 further illustrates that the outer expandable member 506 may include a cavity 508. The cavity 508 may be described as a chamber in which an inflation media (e.g., hydrogel beads, semi-solid materials, thermoreversible copolymers, saline or other suitable liquids, gases, etc.) may be injected (via valve, for example) in order to expand the outer expandable member 506. As an inflation media is inserted into the outer expandable member 506, the inner cavity 508 may expand, thereby permitting the occlusive device 500 to seal against the tissue walls defining an opening in the left atrial appendage.

Inflation of the inner cavity 508 may be accomplished by inserting inflation media through the valve. The valve may include an inflation lumen (not explicitly shown) which may be designed allow the delivery and/or inflation system 550 to be inserted therethrough. The inflation lumen may be aligned with the longitudinal axis of the occlusive member 500.

The delivery and/or inflation system 550 may be similar in form and function to the delivery systems 350, 450 described herein. The delivery and/or inflation system 550 may include an elongate shaft 552 having a lumen 554 extending from a proximal end (not explicitly shown) of the elongate shaft 552 to a distal end of the elongate shaft 552. In some embodiments, the elongate shaft 552 may be a catheter, a hypotube, or other similar tubular structure. In some embodiments, at least a portion of the elongate shaft 552 may include micromachining, a plurality of cuts or weakened areas, some degree of material removal, etc. to provide increased flexibility along a length of the elongate shaft 552 for navigating tortuous vasculature. Some suitable but non-limiting materials for the elongate shaft 552, for example metallic materials, polymer materials, composite materials, etc., are described below. The distal end of the elongate shaft 552 may be configured releasably engage an attachment region 529 of the outer expandable member 506. This may allow the delivery and/or inflation system 550 to be releaseably coupled to the occlusive device 500. In other examples, the attachment region 529 may be designed such that it forms a "press-fit" with a distal end region of a delivery catheter 550. Other methods of attaching the occlusive device 500 to the delivery catheter may include a threaded engagement, a ratcheting mechanism, break-away mechanisms, detent lock, spring lock, single-piece coupling, two-piece coupling, or combinations thereof.

The delivery system 550 may include an intermediate elongate shaft 560 disposed within the lumen 554 of the elongate shaft 552. The intermediate elongate shaft 560 may be a tubular structure, such as but not limited to an elongate shaft, a catheter, a hypotube, or other similar tubular structure. In some embodiments, the intermediate elongate shaft 560 may be slidably disposed within the lumen 554 of the elongate shaft 552. In other embodiments, the intermediate elongate shaft 560 may be fixedly coupled within the lumen 554 of the elongate shaft 552. A lumen 562 may extend from a proximal end (not explicitly shown) of intermediate elongate shaft 560 to a distal end of the intermediate elongate shaft 560. A distal opening may be positioned at the distal end of the intermediate elongate shaft 560.

The delivery and/or inflation system 550 may further include an inner elongate shaft 564 disposed within the lumen 562 of the intermediate elongate shaft 560. The inner elongate shaft 564 may be a tubular structure, such as but not limited to an elongate shaft, a catheter, a hypotube, or other similar tubular structure. In some embodiments, the inner elongate shaft 564 may be slidably disposed within the lumen 562 of the intermediate elongate shaft 560. In other embodiments, the inner elongate shaft 564 may be fixedly coupled within the lumen 562 of the intermediate elongate shaft 560. A lumen 566 may extend from a proximal end (not explicitly shown) of inner elongate shaft 564 to a distal end of the inner elongate shaft 564. The lumen 566 may terminate at a distal opening positioned at the distal end of the inner elongate shaft 564.

While the delivery and/or inflation system 550 is illustrated as a co-axial system, it is contemplated that the elongate shafts 550, 560, 564 may be arranged in such that they extend side-by side as opposed to one within another. Further, the openings for injecting and/or removing the inflation medium may be positioned in a sidewall of the intermediate elongate shaft 560 and/or inner elongated shaft 564, as desired.

A proximal end (not explicitly shown) of the lumen 562 of the intermediate elongate shaft 560 may be coupled to a hub member (not explicitly shown). An inflation media may be passed through the lumen 562 of the intermediate elongate shaft 560 and into the cavity 508 of the outer expandable member 506. In some embodiments, the inflation media may be a thermoreversible copolymer or other media configured to gel or transition to a semi-solid upon delivery, although this is not required.

In some embodiments, the outer expandable member 506 may include a plurality of (e.g., one or more) perfusion apertures 510 extending from an inner surface to an outer surface of the outer expandable member 506. The apertures 510 may be configured to allow an inflation media to weep or leak from the cavity 508 of the outer expandable member 506 and into the LAA 600. When a gelling material (such as, but not limited to, a thermoreversible copolymer) is used, gelation may occur within the cavity 508, through the apertures 510 and into or within the internal LAA 600. This may help to mechanically fixate the occlusive device 500 to and/or within the LAA 600. The apertures 510 may extend over an entire surface of the outer expandable member 506 or along portions thereof. For example, in some embodiments, the apertures 510 may be limited to regions of the outer expandable member 506 expected to be in close proximity to a wall or tissue 602 of the LAA 600. The apertures 510 maybe uniformly or eccentrically arranged, as desired.

In some embodiments, as inflation media seeps from the apertures 510, one or more fixation mechanisms 512 may be deployed. For example, the inflation media may push the one or more fixation mechanisms 512 into the tissue 602 of the LAA 600. In other examples, expansion of the outer expandable member 506 may deploy the fixation mechanisms 512. The fixation mechanisms 512 may include, barbs, hooks, surface texture, bristles, etc. It is contemplated that the fixation mechanism 512 may be used with any of the occlusive devices 10, 100, 300, 400 described herein. Some illustrative fixation mechanisms are described in commonly assigned U.S. Patent Application Number 62/607,053 filed on December 18, 2017 and titled "OCCLUSIVE DEVICE WITH EXPANDABLE MEMBER".

A proximal end (not explicitly shown) of the lumen 566 of the inner elongate shaft 564 may be coupled to a hub member (not explicitly shown). An inflation media may be passed through the lumen 566 of the inner elongate shaft 564 and into the cavity of the inner expandable member 504. It is contemplated that the inflation media may be the same as or different from the inflation media used in the cavity 508 of the outer expandable member 506. A proximal end (not explicitly shown) of the lumen 566 of the inner elongate shaft 564 may be also be configured to be coupled to a hub member or a suction device (not explicitly shown) to remove the inflation material from the cavity of the inner expandable member 504.

In some embodiments, a thermoreversible copolymer inflation media may be injected into the cavity 508 of the outer expandable member 506 and a cooling inflation media or fluid (such as, but not limited to, saline) at a temperature less than the transition temperature of the thermoreversible inflation media may be injected into the cavity of the inner expandable member 504. Cold inflation media may be continuously circulated within the cavity of the inner expandable member 504 to prevent the thermoreversible copolymer inflation media from gelling prematurely. Once the placement of the occlusion deice 500 has been verified, saline (or other inflation media) at body temperature may be circulated into the cavity of the inner expandable member 504 to cause the thermoreversible copolymer inflation media in the cavity 508 of the outer expandable member 506 to gel. Any time prior to uncoupling the delivery system 550 from the occlusive device 500, cold saline (at a temperature less than the transition temperature of the thermoreversible inflation media) may be recirculated into the cavity of the inner expandable member 504 to soften or liquefy the thermoreversible inflation media in the cavity 508 of the outer expandable member 506. This may allow the occlusive member 500 to be repositioned and/or re-formed to the anatomy of the LAA. Further, the inflation media may be removed from both cavities if full recapture is desired and/or necessary.

In some embodiments, the cavity of the inner expandable member 504 may remain filled with the saline inflation media. In other embodiments, the saline may be removed as additional thermoreversible inflation media is injected into the cavity 508 of the outer expandable member 506. The saline may continue to be removed as additional thermoreversible inflation media is injected into the cavity 508 of the outer expandable member 506, until the inner expandable member 504 has collapsed and the occlusive device 500 is filled with thermoreversible inflation media.

FIG. 14 illustrates that the occlusive implant 10 may be inserted and advanced through a body lumen via an occlusive implant delivery system 21. FIG. 14 further illustrates the occlusive implant 10 positioned within the left atrial appendage 60. As discussed above, in some instances the occlusive implant 10 may be positioned within the left atrial appendage such that the nesting region 26 is anchored within a portion of the left atrial appendage 60. While the method and positioning is described with respect to occlusive implant 10, the method may be applicable to any of implants 10, 100, 300, 400, 500 described herein

In some instances, an occlusive implant delivery system 21 may include a delivery catheter 24 which is guided toward the left atrium via various chambers and lumens of the heart (e.g., the inferior vena cava, the superior vena cava, the right atrium, etc.) to a position adjacent the left atrial appendage 60. The delivery system 21 may include a hub member 23 coupled to a proximal region of the delivery catheter 24. The hub member 23 may be manipulated by a clinician to direct the distal end region of the delivery catheter 24 to a position adjacent the left atrial appendage 60. As discussed above, a proximal end of the occlusive device 10 may be configured to releasably attach, join, couple, engage, or otherwise connect to the distal end of the delivery catheter 24. In some embodiments, an end region of the occlusive device 10 may include a threaded insert coupled thereto. In some embodiments, the threaded insert may be configured to and/or adapted to couple with, join to, mate with, or otherwise engage a threaded member disposed at the distal end of the delivery catheter 24. Other means of releasably coupling and/or engaging the proximal end of the occlusive device 10 to the distal end of the delivery catheter are also contemplated. Further, in some examples the delivery catheter 24 may include an inflation lumen (not show) designed to permit inflation media to pass into the occlusive device 10 (as described above). For example, in some examples, the distal end of the delivery catheter 24 may include a needle designed to be inserted through the valve 32 (discussed in FIG. 3).

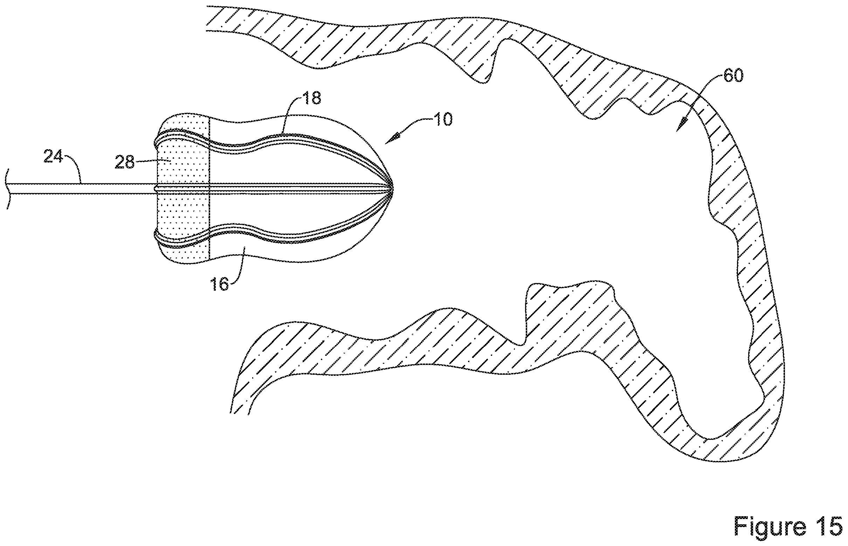

FIGS. 15-17 illustrate the example occlusive device 10 (described above) being positioned and deployed in an opening of the left atrial appendage 60. As discussed above, in some examples, the occlusive device 10 may be configured to shift between a collapsed configuration and an expanded configuration. For example, in some instances, the occlusive implant may be in a collapsed configuration during delivery via an occlusive device delivery system, whereby the occlusive device expands to an expanded configuration once deployed from the occlusion implant delivery system.

FIG. 15 shows the occlusive device 10 including an expandable member 16, a plurality of spine members 18 and a cellular-growth promoting coating 28 (as described above). Further, FIG. 15 illustrates that the occlusive member 10 may be detachably coupled to a delivery catheter 24. The occlusive member 10 shown in FIG. 15 may be described as being in a deflated or delivery configuration. In other words, the expandable member 16 may not contain any inflation media within its inner cavity. It can be appreciated that it may be desirable to maintain the occlusive member 10 in a collapsed configuration when delivering the occlusive member 10 to the target site (e.g., an opening in the left atrial appendage 60). A collapsed configuration may permit the occlusive member 10 to more easily track through tortuous vasculature as a clinician directs the device to the target site.

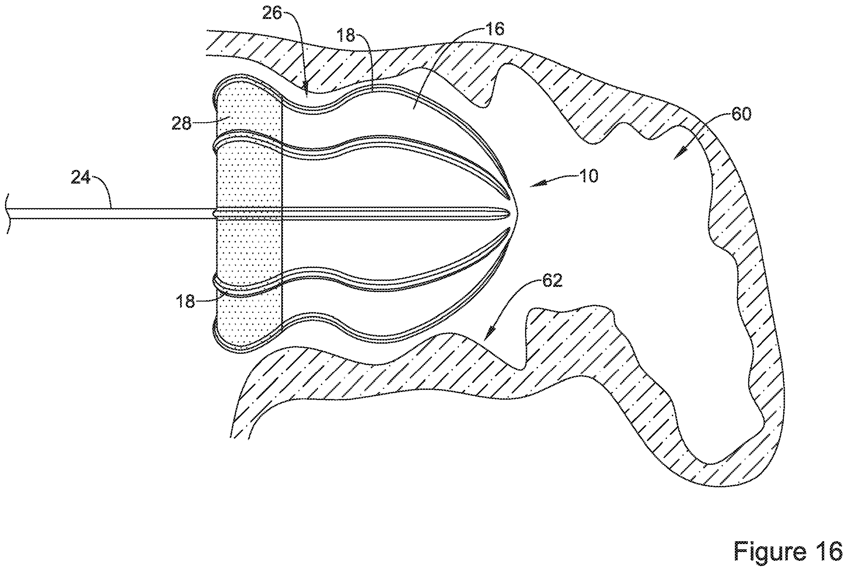

FIG. 16 illustrates an example first stage in deployment of the occlusive member 10. FIG. 16 shows the expandable member 16 expanded to a larger diameter as compared with the non-expanded configuration illustrated in FIG. 15. It can be appreciated that inflation media has been injected into the inner chamber of the expandable member 16, whereby the inflation media shifts the expandable member from the deflated configuration (shown in FIG. 15) to the partially-inflated configuration shown in FIG. 16.

Additionally, FIG. 16 illustrates that as the expandable member 16 inflates radially outward, the spine members 18 approach and may contact the inner surface 62 (e.g., the tissue wall) of the left atrial appendage 60. It can be appreciated that as the spine members 18 (which are circumferentially spaced around the expandable member 16) begin to contact the inner surface 62 of the left atrial appendage 60, they may center and maintain the occlusive device 10 within the opening of the left atrial appendage 60. Additionally, as the spine members 18 contact the inner surface 62 of the atrial appendage 60 they may reduce the likelihood that occlusive device 10 will shift its position within the left atrial appendage 60. Additionally, when aligned properly, the nesting region 26 of the occlusive member 10 may nest within a portion of the wall of the left atrial appendage 60, thereby furthering reducing the likelihood that the occlusive member 10 will shift its position while in the partially deflated state shown in FIG. 16.

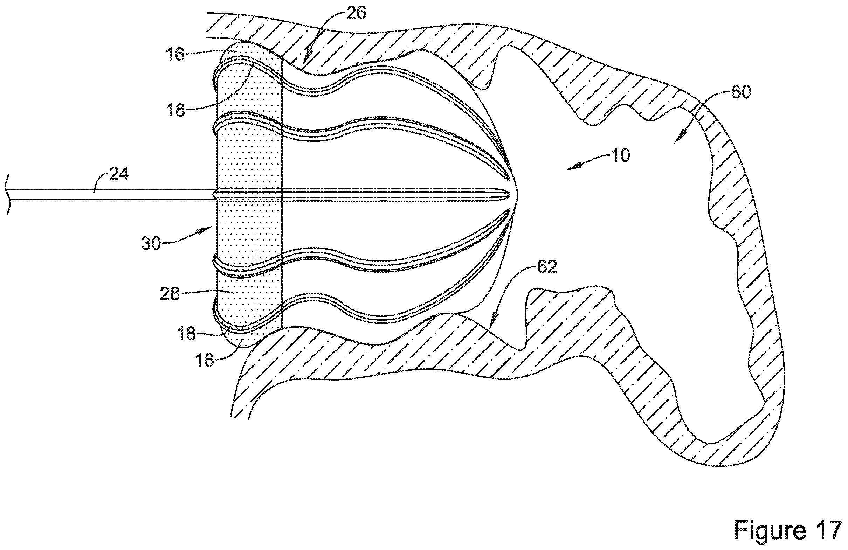

FIG. 17 illustrates the occlusive member 10 in a fully inflated state. Additionally, FIG. 17 illustrates that the expandable member 16 may be compliant and, therefore, substantially conform to and/or be in sealing engagement with the shape and/or geometry of a lateral wall 62 of a left atrial appendage 60 while in the inflated (e.g., expanded) configuration. In some embodiments, the occlusive device 10 may expand to a size, extent, or shape different from a maximum unconstrained extent, as determined by the surrounding tissue and/or lateral wall 62 of the left atrial appendage 60.

As can be appreciated from FIG. 17, continued inflation of the expandable member 16 beyond the partially inflated state shown in FIG. 16 may permit the expandable member 16 to expand and conform to the specific geometry of the inner surface 62 of the left atrial appendage 60. In other words, as inflation media is added to the expandable member 16, the expandable member 16 may fill and/or seal gaps in the opening of the left atrial appendage 60 which may not have been sealed while the occlusive device 10 was partially inflated (as shown in FIG. 16). It can be appreciated that the flexible material used to construct the expandable member 16 may stretch, conform and directly oppose the folded curvature of the inner surface 62 of the left atrial appendage 60. For example, FIG. 17 shows the expandable member 16 expanded such that the expandable member 16 is contacting the curved inner surface 62 of the left atrial appendage 60, thereby sealing the opening of the left atrial appendage 60. Additionally, FIG. 17 illustrates the nesting region 26 of the occlusive member seated within a portion of the inner surface 62 of the left atrial appendage 60.

It can further be appreciated from FIG. 17 that the bottom surface 30 of the occlusive device is positioned such that it is facing the left atrium of the heart. As discussed above, the bottom surface 30 of the occlusive device 10 may include the cellular-growth promoting coating 28. Accordingly, the cellular-growth promoting coating 28 is positioned to promote the growth of endothelial cellular tissue across the bottom surface 30 of the occlusive implant 10, thereby effectively sealing the left atrial appendage 60.

FIG. 18 and FIG. 19 show cross-sectional views of the occlusive device 10 being inflated from a partially-inflated state (shown in FIG. 16) to a fully inflated state (shown in FIG. 17.) whereby the expandable member 16 fully opposes the inner surface 62 of the left atrial appendage 60. FIG. 18 further illustrates a delivery catheter 24 (described above in some examples as a secondary medical device) having been advanced through the lumen 36 of the valve 32. As described above, the O-ring 38 has expanded radially outward to permit the distal end region of the delivery catheter 24 to be advanced through the valve lumen 36 and into the inner chamber 34 of the expandable member 16. Once positioned within the inner chamber 34, the inflation media (depicted by the arrows in FIG. 18) may be injected into the inner chamber 34, thereby expanding the occlusive device 10 as described above.

FIG. 19 shows the occlusive device 10 deployed along the inner surface 62 of the left atrial appendage 60. Further, FIG. 19 illustrates the delivery catheter 24 described above in FIG. 18 having been removed from the inflation lumen 36 of the valve 32. It can be appreciated from FIG. 19 that the O-ring 38 has been compressed radially inward such that it has closed the lumen 36. It can be further appreciated that the O-ring 38 may designed to exert sufficient radially inward force along the valve 36 to prevent the inflation media from passing back through the valve 32 (which may partially collapse the occlusive device 10).

The materials that can be used for the various components of the occlusive implant 10 (and variations, systems or components thereof disclosed herein) and the various elements thereof disclosed herein may include those commonly associated with medical devices. For simplicity purposes, the following discussion makes reference to the occlusive implant 10 (and variations, systems or components disclosed herein). However, this is not intended to limit the devices and methods described herein, as the discussion may be applied to other elements, members, components, or devices disclosed herein.

In some embodiments, the occlusive implant 10 (and variations, systems or components thereof disclosed herein) may be made from a metal, metal alloy, polymer (some examples of which are disclosed below), a metal-polymer composite, ceramics, combinations thereof, and the like, or other suitable material. Some examples of suitable metals and metal alloys include stainless steel, such as 444V, 444L, and 314LV stainless steel; mild steel; nickel-titanium alloy such as linear-elastic and/or super-elastic nitinol; other nickel alloys such as nickel-chromium-molybdenum alloys (e.g., UNS: N06625 such as INCONEL^{®} 625, UNS: N06022 such as HASTELLOY^{®} C-22^{®}, UNS: N10276 such as HASTELLOY^{®} C276^{®}, other HASTELLOY^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R44035 such as MP35-N^{®} and the like), nickel-molybdenum alloys (e.g., UNS: N10665 such as HASTELLOY^{®} ALLOY B2^{®}), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R44003 such as ELGILOY^{®}, PHYNOX^{®}, and the like); platinum enriched stainless steel; titanium; platinum; palladium; gold; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear than the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also be distinguished based on its composition), which may accept only about 0.2 to 0.44 percent strain before plastically deforming.

In some embodiments, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Other suitable materials may include ULTANIUM^{™} (available from Neo-Metrics) and GUM METAL^{™} (available from Toyota). In some other embodiments, a superelastic alloy, for example a superelastic nitinol can be used to achieve desired properties.

In at least some embodiments, portions or all of the occlusive implant 10 (and variations, systems or components thereof disclosed herein) may also be doped with, made of, or otherwise include a radiopaque material. Radiopaque materials are understood to be materials capable of producing a relatively bright image on a fluoroscopy screen or another imaging technique during a medical procedure. This relatively bright image aids a user in determining the location of the occlusive implant 10 (and variations, systems or components thereof disclosed herein). Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of the occlusive implant 10 (and variations, systems or components thereof disclosed herein) to achieve the same result.

In some embodiments, a degree of Magnetic Resonance Imaging (MRI) compatibility is imparted into the occlusive implant 10 (and variations, systems or components thereof disclosed herein). For example, the occlusive implant 10 (and variations, systems or components thereof disclosed herein) and/or components or portions thereof, may be made of a material that does not substantially distort the image and create substantial artifacts (e.g., gaps in the image). Certain ferromagnetic materials, for example, may not be suitable because they may create artifacts in an MRI image. The occlusive implant 10 (and variations, systems or components disclosed herein) or portions thereof, may also be made from a material that the MRI machine can image. Some materials that exhibit these characteristics include, for example, tungsten, cobalt-chromium-molybdenum alloys (e.g., UNS: R44003 such as ELGILOY^{®}, PHYNOX^{®}, and the like), nickel-cobalt-chromium-molybdenum alloys (e.g., UNS: R44035 such as MP35-N^{®} and the like), nitinol, and the like, and others.

In some embodiments, the occlusive implant 10 (and variations, systems or components thereof disclosed herein) and/or portions thereof, may be made from or include a polymer or other suitable material. Some examples of suitable polymers may include copolymers, polyisobutylene-polyurethane, polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), Marlex high-density polyethylene, Marlex low-density polyethylene, linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR^{®}), polysulfone, nylon, nylon-12 (such as GRILAMID^{®} available from EMS American Grilon), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS 50A), polycarbonates, ionomers, polyurethane silicone copolymers (for example, ElastEon^{®} from Aortech Biomaterials or ChronoSil^{®} from AdvanSource Biomaterials), biocompatible polymers, other suitable materials, or mixtures, combinations, copolymers thereof, polymer/metal composites, and the like. In some embodiments, the sheath can be blended with a liquid crystal polymer (LCP). For example, the mixture can contain up to about 6 percent LCP.

In some embodiments, the occlusive implant 10 (and variations, systems or components thereof disclosed herein) may include a textile material. Some examples of suitable textile materials may include synthetic yarns that may be flat, shaped, twisted, textured, pre-shrunk or un-shrunk. Synthetic biocompatible yarns suitable for use in the present disclosure include, but are not limited to, polyesters, including polyethylene terephthalate (PET) polyesters, polypropylenes, polyethylenes, polyurethanes, polyolefins, polyvinyls, polymethylacetates, polyamides, naphthalene dicarboxylene derivatives, natural silk, and polytetrafluoroethylenes. Moreover, at least one of the synthetic yarns may be a metallic yarn or a glass or ceramic yarn or fiber. Useful metallic yarns include those yarns made from or containing stainless steel, platinum, gold, titanium, tantalum or a Ni-Co-Cr-based alloy. The yarns may further include carbon, glass or ceramic fibers. Desirably, the yarns are made from thermoplastic materials including, but not limited to, polyesters, polypropylenes, polyethylenes, polyurethanes, polynaphthalenes, polytetrafluoroethylenes, and the like. The yarns may be of the multifilament, monofilament, or spun-types. The type and denier of the yarn chosen may be selected in a manner which forms a biocompatible and implantable prosthesis and, more particularly, a vascular structure having desirable properties.

In some embodiments, the occlusive implant 10 (and variations, systems or components thereof disclosed herein) may include and/or be treated with a suitable therapeutic agent. Some examples of suitable therapeutic agents may include anti-thrombogenic agents (such as heparin, heparin derivatives, urokinase, and PPack (dextrophenylalanine proline arginine chloromethylketone)); anti-proliferative agents (such as enoxaparin, angiopeptin, monoclonal antibodies capable of blocking smooth muscle cell proliferation, hirudin, and acetylsalicylic acid); anti-inflammatory agents (such as dexamethasone, prednisolone, corticosterone, budesonide, estrogen, sulfasalazine, and mesalamine); antineoplastic/antiproliferative/anti-mitotic agents (such as paclitaxel, 5-fluorouracil, cisplatin, vinblastine, vincristine, epothilones, endostatin, angiostatin and thymidine kinase inhibitors); anesthetic agents (such as lidocaine, bupivacaine, and ropivacaine); anti-coagulants (such as D-Phe-Pro-Arg chloromethyl ketone, an RGD peptide-containing compound, heparin, anti-thrombin compounds, platelet receptor antagonists, anti-thrombin antibodies, anti-platelet receptor antibodies, aspirin, prostaglandin inhibitors, platelet inhibitors, and tick antiplatelet peptides); vascular cell growth promoters (such as growth factor inhibitors, growth factor receptor antagonists, transcriptional activators, and translational promoters); vascular cell growth inhibitors (such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin); cholesterol-lowering agents; vasodilating agents; and agents which interfere with endogenous vascoactive mechanisms.

While the discussion above is generally directed toward an occlusive implant for use in the left atrial appendage of the heart, the aforementioned features may also be useful in other types of medical implants where a fabric or membrane is attached to a frame or support structure including, but not limited to, implants for the treatment of aneurysms (e.g., abdominal aortic aneurysms, thoracic aortic aneurysms, etc.), replacement valve implants (e.g., replacement heart valve implants, replacement aortic valve implants, replacement mitral valve implants, replacement vascular valve implants, etc.), and/or other types of occlusive devices (e.g., atrial septal occluders, cerebral aneurysm occluders, peripheral artery occluders, etc.). Other useful applications of the disclosed features are also contemplated.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The disclosure's scope is, of course, defined in the language in which the appended claims are expressed.

## Claims

1. A medical device for occluding a left atrial appendage (60), comprising:
an expandable member (302, 402, 502) having a first end region (312, 412, 513) and a second end region (314, 414, 515), the expandable member comprising at least one inflation cavity (308, 334, 408, 434, 508) and at least one valve member (322, 332, 422, 432) configured to selectively seal the at least one inflation cavity;
a first inflation media (370, 468) disposed within the at least one inflation cavity;
a second inflation media (368, 474) disposed within the at least one inflation cavity, the second inflation media different than the first inflation media;
wherein the expandable member is configured to expand and seal the opening of the left atrial appendage,
wherein the expandable member comprises:
an inner expandable member (304, 404, 504) having an inner inflation cavity (334, 434) and a first valve member (332, 432) configured to selectively seal the inner inflation cavity (334); and
an outer expandable member (306, 406, 506) having an outer inflation cavity (308, 408, 508) and a second valve member (322, 422) configured to selectively seal the outer inflation cavity the inner expandable member being disposed within the outer inflation cavity,
wherein a plurality of apertures (309) extend from an inner surface (305) to an outer surface (307) of the outer expandable member (306, 406, 306), and
wherein the outer expandable member (306, 406, 506) is configured to receive the first inflation media (370, 468) and the inner expandable member (304, 404, 504) is configured to receive the second inflation media (368, 474)

2. The medical device of claim 1, wherein the first inflation media (370, 468) is a thermally reversible copolymer.

3. The medical device of claim 2, wherein the thermally reversible copolymer is a liquid at temperatures in the range of about 20 to about 25 °C and a gel at temperatures in the range of about 36 to about 37.5 °C.

4. The medical device of any one of claims 2-3,
wherein the second inflation media (368, 474) is delivered at a temperature less than the transition temperature of the thermally reversible copolymer.

5. The medical device of any one of claims 2-4, wherein the thermally reversible copolymer is a polyethylene glycol-poly(lactic-co-glycolic acid)-polyethylene glycol or a polyethylene glycol-polycaprolactone-polyethylene glycol.

6. The medical device of any one of claims 1-5, wherein an inflation media (370) is configured to weep from the plurality of apertures.

## Patentansprüche

1. Medizinische Vorrichtung zum Verschließen eines linken Vorhofohrs (60), mit:
einem expandierbaren Element (302, 402, 502) mit einem ersten Endbereich (312, 412, 513) und einem zweiten Endbereich (314, 414, 515), wobei das expandierbare Element mindestens einen Aufpumphohlraum (308, 334, 408, 434, 508) und mindestens ein Ventilelement (322, 332, 422, 432) aufweist, das dafür konfiguriert ist, den mindestens einen Aufpumphohlraum selektiv abzudichten;
einem ersten Aufpumpmedium (370, 468), das in dem mindestens einen Aufpumphohlraum angeordnet ist;
einem zweiten Aufpumpmedium (368, 474), das in dem mindestens einen Aufpumphohlraum angeordnet ist, wobei sich das zweite Aufpumpmedium vom ersten Aufpumpmedium unterscheidet,
wobei das expandierbare Element dafür konfiguriert ist, die Öffnung des linken Vorhofohrs zu dehnen und abzudichten,
wobei das expandierbare Element aufweist:
ein inneres expandierbares Element (304, 404, 504) mit einem inneren Aufpumphohlraum (334, 434) und einem ersten Ventilelement (332, 432), das dafür konfiguriert ist, den inneren Aufpumphohlraum (334) selektiv abzudichten; und
ein äußeres expandierbares Element (306, 406, 506) mit einem äußeren Aufpumphohlraum (308, 408, 508) und einem zweiten Ventilelement (322, 422), das dafür konfiguriert ist, den äußeren Aufpumphohlraum selektiv abzudichten, wobei das innere ausdehnbare Element innerhalb des äußeren Aufpumphohlraums angeordnet ist,
wobei sich eine Vielzahl von Öffnungen (309) von einer Innenfläche (305) zu einer Außenfläche (307) des äußeren expandierbaren Elements (306, 406, 506) erstrecken, und
wobei das äußere expandierbare Element (306, 406, 506) dafür konfiguriert ist, das erste Aufpumpmedium (370, 468) aufzunehmen, und das innere expandierbare Element (304, 404, 504) dafür konfiguriert ist, das zweite Aufpumpmedium (368, 474) aufzunehmen.

2. Medizinische Vorrichtung nach Anspruch 1, wobei das erste Aufpumpmedium (370, 468) ein thermisch reversibles Copolymer ist.

3. Medizinische Vorrichtung nach Anspruch 2, wobei das thermisch reversible Copolymer bei Temperaturen im Bereich von etwa 20 bis etwa 25°C eine Flüssigkeit und bei Temperaturen im Bereich von etwa 36 bis etwa 37,5 °C ein Gel ist.

4. Medizinische Vorrichtung nach Anspruch 2 oder 3, wobei das zweite Aufpumpmedium (368, 474) bei einer Temperatur abgegeben wird, die niedriger ist als die Übergangstemperatur des thermisch reversiblen Copolymers.

5. Medizinische Vorrichtung nach einem der Ansprüche 2 bis 4, wobei das thermisch reversible Copolymer ein Polyethylenglykol-Poly(milch-co-glykolsäure)-Polyethylenglykol oder ein Polyethylenglykol-Polycaprolacton-Polyethylenglykol ist.

6. Medizinische Vorrichtung nach einem der Ansprüche 1 bis 5, wobei ein Aufpumpmedium (370) derart konfiguriert ist, dass es aus der Vielzahl von Öffnungen auströpfelt.

## Revendications

1. Dispositif médical pour fermer un appendice auriculaire gauche (60), comprenant :
un élément extensible (302, 402, 502) ayant une première région d'extrémité (312, 412, 513) et une deuxième région d'extrémité (314, 414, 515), l'élément extensible comprenant au moins une cavité de gonflage (308, 334, 408, 434, 508) et au moins un élément de valve (322, 332, 422, 432) configuré pour sceller sélectivement la ou les cavités de gonflage ;
un premier milieu de gonflage (370, 468) disposé à l'intérieur de l'au moins une cavité de gonflage ;
un deuxième milieu de gonflage (368, 474) disposé à l'intérieur de l'au moins une cavité de gonflage, le deuxième milieu de gonflage étant différent du premier milieu de gonflage ;
dans lequel l'élément extensible est configuré pour dilater et sceller l'ouverture de l'appendice auriculaire gauche,
dans lequel l'élément extensible comprend :
un élément extensible interne (304, 404, 504) comportant une cavité de gonflage interne (334, 434) et un premier élément de valve (332, 432) configuré pour sceller sélectivement la cavité de gonflage interne (334) ; et
un élément extensible externe (306, 406, 506) comportant une cavité de gonflage externe (308, 408, 508) et un deuxième élément de valve (322, 422) configuré pour sceller sélectivement la cavité de gonflage externe, l'élément interne extensible étant disposé à l'intérieur de la cavité de gonflage externe, dans lequel une pluralité d'ouvertures (309) s'étendent d'une surface interne (305) à une surface externe (307) de l'élément extensible externe (306, 406, 306), et
dans lequel l'élément extensible externe (306, 406, 506) est configuré pour recevoir le premier milieu de gonflage (370, 468) et l'élément interne extensible (304, 404, 504) est configuré pour recevoir le deuxième milieu de gonflage (368, 474).

2. Dispositif médical selon la revendication 1, dans lequel le premier milieu de gonflage (370, 468) est un copolymère thermiquement réversible.

3. Dispositif médical selon la revendication 2, dans lequel le copolymère thermiquement réversible est un liquide à des températures comprises dans la plage d'environ 20 à environ 25 °C et un gel à des températures dans la plage d'environ 36 à environ 37,5 °C.

4. Dispositif médical selon la revendication 2 ou la revendication 3, dans lequel le deuxième milieu de gonflage (368, 474) est délivré à une température inférieure à la température de transition du copolymère thermiquement réversible.

5. Dispositif médical selon l'une quelconque des revendications 2 à 4, dans lequel le copolymère thermiquement réversible est un polyéthylène glycol-poly(acide lactique-co-glycolique)-polyéthylène glycol ou un polyéthylène glycol-polycaprolactone-polyéthylène glycol.

6. Dispositif médical selon l'une quelconque des revendications 1 à 5, dans lequel un milieu de gonflage (370) est configuré pour s'échapper de la pluralité d'ouvertures.
